# EUROPEAN PATENT SPECIFICATION

(11) **EP 3 397 281 B1**
(45) Date of publication and mention of the grant of the patent: **09.09.2020**
(21) Application number: 16826865.4
(22) Date of filing: 28.12.2016
(51) Int. Cl.: A61K 39/395, C07K 16/22, C07K 16/24, A61P 35/00

(54) **BUFFERED FORMULATIONS OF BEVACIZUMAB**
GEPUFFERTE FORMULIERUNGEN VON BEVACIZUMAB
FORMULATIONS TAMPONNÉES DE BÉVACIZUMAB

(30) Priority: 29.12.2015 US 201562272116 P
(43) Date of publication of application: 07.11.2018
(73) Proprietor: Outlook Therapeutics, Inc., Cranbury, NJ 08512 (US)
(72) Inventor: TADDEI, Maria, Cranbury New Jersey 08512 (US); CHEUNG, Jessica, Cranbury New Jersey 08512 (US); GUTKA, Hiten, Cranbury New Jersey 08512 (US)
(74) Representative: Cooley (UK) LLP
(86) International application number: PCT/US2016/068847
(87) International publication number: WO 2017/117202

(56) References cited:
- M. R. STOCKLER ET AL: "Patient-Reported Outcome Results From the Open-Label Phase III AURELIA Trial Evaluating Bevacizumab-Containing Therapy for Platinum-Resistant Ovarian Cancer", JOURNAL OF CLINICAL ONCOLOGY, vol. 32, no. 13, 31 March 2014 (2014-03-31), pages 1309-1316, XP055349146, US ISSN: 0732-183X, DOI: 10.1200/JCO.2013.51.4240
- NICHOLAS W WARNE ED - LEHR CLAUS-MICHAEL ET AL: "Development of high concentration protein biopharmaceuticals: The use of platform approaches in formulation development", EUROPEAN JOURNAL OF PHARMACEUTICS AND BIOPHARMACEUTICS, ELSEVIER SCIENCE PUBLISHERS B.V., AMSTERDAM, NL, vol. 78, no. 2, 3 March 2011 (2011-03-03), pages 208-212, XP028203394, ISSN: 0939-6411, DOI: 10.1016/J.EJPB.2011.03.004 [retrieved on 2011-03-13] & US 2015/071925 A1 (LARSON ALYSSA M [US] ET AL) 12 March 2015 (2015-03-12)
- Robert L Garnick ET AL: "ATTACHMENT D AVASTIN~~ Approval Letter DEPARIMENT OF HEALTH & HUMAN SERVICES Public Health Service", , 1 September 2004 (2004-09-01), XP055349240, Retrieved from the Internet: URL:https://www.fda.gov/OHRMS/dockets/dail ys/04/sep04/090304/04e-0402-app0001-05-Att achment-D-Avastin-Approval-Letter-vol1.pdf [retrieved on 2017-02-24]
- MARIEKE VEURINK ET AL: "Association of ranibizumab (Lucentis) or bevacizumab (Avastin) with dexamethasone and triamcinolone acetonide: Anstability assessment", EUROPEAN JOURNAL OF PHARMACEUTICS AND BIOPHARMACEUTICS, vol. 78, no. 2, 2011, pages 271-277, XP028203401, ISSN: 0939-6411, DOI: 10.1016/J.EJPB.2010.12.018 [retrieved on 2010-12-21]
- YATIN R GOKARN ET AL: "Self-buffering antibody formulations", JOURNAL OF PHARMACEUTICAL SCIENCES, AMERICAN PHARMACEUTICAL ASSOCIATION, WASHINGTON, US, vol. 97, no. 8, 1 August 2008 (2008-08-01) , pages 3051-3066, XP002638374, ISSN: 0022-3549, DOI: 10.1002/JPS.21232 [retrieved on 2007-11-19]
- WANG W ET AL: "ANTIBODY STRUCTURE, INSTABILITY, AND FORMULATION", JOURNAL OF PHARMACEUTICAL SCIENCES, AMERICAN PHARMACEUTICAL ASSOCIATION, WASHINGTON, US, vol. 96, no. 1, 1 January 2007 (2007-01-01), pages 1-26, XP009084505, ISSN: 0022-3549, DOI: 10.1002/JPS.20727

## Description

### FIELD OF THE INVENTION

The present disclosure relates generally to the field of antibody formulation chemistry. More particularly, the invention relates to buffered formulations of an antibody to vascular endothelial growth factor, as defined in the appended claims, which formulations enhance the thermal stability and colloidal stability of the antibody, thereby enhancing long-term storage of the antibody.

### BACKGROUND OF THE INVENTION

As part of the Biologics Price Competition and Innovation Act (BPCIA), a biological drug product (produced in or derived from living organisms) may be demonstrated to be "biosimilar" if data show that, among other things, the product is "highly similar" to an already-approved biological product. The biosimilar product should retain at least the biologic function and treatment efficacy of the U.S. Food and Drug Agency-approved biological product. The biosimilar product can be formulated differently, however, from the approved biological product. The formulation can improve stability and shelf storage of the biologic drug product, and can also improve the efficacy in treating a particular disease or condition. The formulation can also improve other aspects of administration, including a reduction in patient discomfort or other untoward effects that a patient may experience upon administration of the approved biological product.

Antibody molecules can be used as biological drugs, and many such antibodies are approved for use in human beings. Antibody molecules can be produced as a biosimilar, and reformulated accordingly. There remains a need in the art for high quality antibody biosimilars.

The bevacizumab antibody marketed under the brand Avastin® (Genentech, Inc., San Francisco, CA) is known to aggregate in two forms under storage conditions - a non-covalent, reversible aggregate and a covalent, non-reversible aggregate. It is believed that the latter (covalent aggregate) occurs in the antigen-binding domain and, therefore, reduces the number of binding sites available to bind to vascular endothelial growth factor (VEGF). As a result, the potency of the antibody is diminished. Reduction of such aggregates is desirable generally, and particularly for an antibody such as bevacizumab. N W Warne, Eur J Pharm Biopharm, 2011 Jun;78(2):208-12, discloses a Bevacizumab formulation at a concentration of 25 mg/ml antibody with 60 mg/mL trehalose, 5.8 mg/mL sodium phosphate monobasic monohydrate, 1.2 mg/mL sodium phosphate dibasic anhydrous, 0.4 mg/mL polysorbate-20, and a pH of 6.2. The present disclosure addresses these needs in the art.

### SUMMARY OF THE INVENTION

The invention features buffered formulations for storage of bevacizumab, as defined in claim 1. The bevacizumab comprises a heavy chain comprising the amino acid sequence of SEQ ID NO: 1 and a light chain comprising the amino acid sequence of SEQ ID NO: 2. The bevacizumab comprises a heavy chain variable region comprising the amino acid sequence of SEQ ID NO: 3 and a light chain variable region comprising the amino acid sequence of SEQ ID NO: 4.
In the buffered formulation of the invention, bevacizumab is present in a concentration of from 15 mg/ml to 35 mg/ml, or more preferably from 24 mg/ml to 27 mg/ml, or more preferably 25 mg/ml or 25.5 mg/ml.
In an example disclosed for reference, bevacizumab may be present in a concentration of from about 10 mg to about 50 mg.
Also disclosed are examples wherein the formulation is aqueous, and the buffer may comprise citrate phosphate or sodium acetate, and the formulation may also comprise a stabilizer that comprises a sugar such as trehalose or sucrose, as well as a mild surfactant such as polysorbate 20.
In the embodiments of the invention, the formulation has a pH of 5.6 to 5.8, and in some aspects, the pH is 5.6 or 5.8. Also disclosed are examples wherein the formulation preferably has an acidic pH of from about 5.6 to about 6.1, and in some aspects, the pH is about 5.6, or about 5.8, or about 6.

In some examples, the formulation comprises a buffer comprising from about 10 mM to about 100 mM of citrate phosphate, from about 100 mM to about 200 mM of trehalose, and from about 0.01% (v/v) to about 0.1% (v/v) of polysorbate 20, and has a pH of from about 5.7 to about 6.1. The citrate phosphate may be at a concentration range of from about 30 mM to about 70 mM, from about 40 mM to about 60 mM, from about 48 mM to about 52 mM, from about 49 mM to about 51 mM, or from about 50 mM to about 51 mM, or may be at a concentration of about 50 mM or about 51 mM. The trehalose may be at a concentration range of from about 120 mM to about 180 mM, from about 150 mM to about 170 mM, from about 157 mM to about 161 mM, from about 140 mM to about 180 mM, or from about 158 mM to about 160 mM, or at a concentration of about 159 mM or about 160 mM. The polysorbate may be at a concentration range of from about 0.02% (v/v) to about 0.06% (v/v), or from about 0.03% (v/v) to about 0.05% (v/v), or may be at a concentration of about 0.04% (v/v). The formulation pH may be about 5.8 or may be about 6.

In some aspects of the invention, the formulation comprises a buffer comprising from 5 mM to 25 mM of sodium acetate trihydrate, from 150 mM to 201 mM of sucrose, and from 0.03% (v/v) to 0.05% (v/v) of polysorbate 20, and has a pH of from 5.6 to about 5.8. The sodium acetate trihydrate may be at a concentration range of from 11 mM to 19 mM, from 13 mM to 17 mM, or from 13 mM to 16 mM, or may be at a concentration of 15 mM. The sucrose may be at a concentration range of from 165 mM to 185 mM, from 170 mM to 180 mM, or from 174 mM to 176 mM, or may be at a concentration of 175 mM.

The present disclosure provides a buffered antibody formulation, comprising an antibody comprising a heavy chain comprising the amino acid sequence of SEQ ID NO: 1 and a light chain comprising the amino acid sequence of SEQ ID NO: 2, a buffer comprising from about 10 mM to about 100 mM of citrate phosphate, from about 100 mM to about 200 mM of trehalose, and from about 0.01% (v/v) to about 0.1% (v/v) of polysorbate 20, wherein the antibody formulation has a pH of from about 5.7 to about 6.1. Preferably, the antibody formulation is stable for at least 18 months when stored under refrigerated conditions at 5°C.

The present disclosure also provides a buffered antibody formulation, comprising from about 15 mg/ml to about 35 mg/ml of an antibody comprising a heavy chain comprising the amino acid sequence of SEQ ID NO: 1 and a light chain comprising the amino acid sequence of SEQ ID NO: 2, a buffer comprising from about 40 mM to about 60 mM of citrate phosphate, from about 140 mM to about 180 mM of trehalose, and from about 0.02% (v/v) to about 0.06% (v/v) of polysorbate 20, wherein the antibody formulation has a pH of from about 5.7 to about 6.1. Preferably, the antibody formulation is stable for at least 18 months when stored under refrigerated conditions at 5°C.

The present disclosure provides a buffered antibody formulation, comprising from about 24 mg/ml to about 27 mg/ml of an antibody comprising a heavy chain comprising the amino acid sequence of SEQ ID NO: 1 and a light chain comprising the amino acid sequence of SEQ ID NO: 2, a buffer comprising from about 48 mM to about 52 mM of citrate phosphate, from about 157 mM to about 161 mM of trehalose, and from about 0.03% (v/v) to about 0.05% (v/v) of polysorbate 20, wherein the antibody formulation has a pH of from about 5.8 to about 6.0. Preferably, the antibody formulation is stable for at least 18 months when stored under refrigerated conditions at 5°C.

The present disclosure provides a buffered antibody formulation, comprising an antibody comprising a heavy chain comprising the amino acid sequence of SEQ ID NO: 1 and a light chain comprising the amino acid sequence of SEQ ID NO: 2, a buffer comprising about 50 mM of citrate phosphate, about 159 mM of trehalose, and about 0.04% (v/v) of polysorbate 20, wherein the antibody formulation has a pH of about 5.8 or about 6. Preferably, the antibody formulation is stable for at least 18 months when stored under refrigerated conditions at 5°C.

The present disclosure also provides a buffered antibody formulation, comprising from about 20 mg/ml to about 30 mg/ml of an antibody comprising a heavy chain comprising the amino acid sequence of SEQ ID NO: 1 and a light chain comprising the amino acid sequence of SEQ ID NO: 2, a buffer comprising from about 5 mM to about 25 mM of sodium acetate, from about 150 mM to about 201 mM of sucrose, and from about 0.03% (v/v) to about 0.05% (v/v) of polysorbate 20, wherein the antibody formulation has a pH of from about 5.6 to about 5.8. Preferably, the antibody formulation is stable for at least 18 months when stored under refrigerated conditions at 5°C.

The present disclosure also provides a buffered antibody formulation, comprising from about 24 mg/ml to about 26 mg/ml of an antibody comprising a heavy chain comprising the amino acid sequence of SEQ ID NO: 1 and a light chain comprising the amino acid sequence of SEQ ID NO: 2, a buffer comprising from about 13 mM to about 17 mM of sodium acetate, from about 170 mM to about 180 mM of sucrose, and from about 0.03% (v/v) to about 0.05% (v/v) of polysorbate 20, wherein the antibody formulation has a pH of from about 5.6 to about 5.8. Preferably, the antibody formulation is stable for at least 18 months when stored under refrigerated conditions at 5°C.

In an embodiment, the present invention also provides a buffered antibody formulation, comprising an antibody comprising a heavy chain comprising the amino acid sequence of SEQ ID NO: 1 and a light chain comprising the amino acid sequence of SEQ ID NO: 2, a buffer comprising about 15 mM of sodium acetate, about 175 mM of sucrose, and about 0.04% (v/v) of polysorbate 20, wherein the antibody formulation has a pH of about 5.6 or about 5.8, wherein the antibody formulation is stable for at least 18 months when stored under refrigerated conditions at 5°C.

In an embodiment, the present invention also provides a buffered antibody formulation, comprising an antibody comprising a heavy chain comprising the amino acid sequence of SEQ ID NO: 1 and a light chain comprising the amino acid sequence of SEQ ID NO: 2, a buffer comprising about 15 mM of sodium acetate, about 175 mM of sucrose, and about 0.04% (v/v) of polysorbate 20, wherein the antibody formulation has a pH of about 5.6 or about 5.8, wherein the antibody formulation is stable for at least 18 months when stored under refrigerated conditions at 5°C.

The present disclosure also provides a kit comprising any buffered antibody formulation, comprising an antibody comprising a heavy chain comprising the amino acid sequence of SEQ ID NO: 1 and a light chain comprising the amino acid sequence of SEQ ID NO: 2 disclosed herein. The kit can further comprise a device for injecting the antibody formulation into a subject. The device can comprise a syringe, a needle, a catheter, or any combination thereof. The kit can further comprise instructions for treating one or more of the cancers disclosed herein.

The present disclosure also provides methods for treating cancer in a subject in need thereof, the method comprising administering to the subject any buffered antibody formulation, comprising an antibody comprising a heavy chain comprising the amino acid sequence of SEQ ID NO: 1 and a light chain comprising the amino acid sequence of SEQ ID NO: 2 disclosed herein in an amount effective to treat said cancer.

The present disclosure also provides any buffered antibody formulation, comprising an antibody comprising a heavy chain comprising the amino acid sequence of SEQ ID NO: 1 and a light chain comprising the amino acid sequence of SEQ ID NO: 2 disclosed herein for use in the manufacture of a medicament for the treatment of cancer.

Any of the antibody formulations can used in a method for treating one or more of platinum-resistant recurrent epithelial ovarian, fallopian tube, or primary peritoneal cancer. In general, the methods comprise administering the formulation, including the bevacizumab antibody, to a subject in need thereof, in an amount effective to treat one or more of platinum-resistant recurrent epithelial ovarian, fallopian tube, or primary peritoneal cancer. The subject is preferably a human being, and the formulation is preferably administered via intravenous infusion or injection. Any of the antibody formulations may similarly be used in the manufacture of a medicament for the treatment of cancer such as one or more of platinum-resistant recurrent epithelial ovarian, fallopian tube, or primary peritoneal cancer, persistent, recurrent or metastatic cervical cancer, metastatic colorectal cancer, metastatic HER2 (human epidermal growth factor receptor 2) negative breast cancer, metastatic renal cell carcinoma, glioblastoma, or non-small cell lung cancer.

Unless defined otherwise, all technical and scientific terms used herein have the same meaning as commonly understood by one of ordinary skill in the art to which this invention belongs.

### BRIEF DESCRIPTION OF THE DRAWINGS

Figure 1 shows a DSC plot showing the effect of various stabilizers on bevacizumab thermal stability in a 50 mM sodium phosphate buffer. Conditions 1, 2, 9, and 10 from Table 1 are shown in the plot.
Figure 2A shows the percent of bevacizumab aggregates in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when stored at 5 °C over a duration of 18 months.

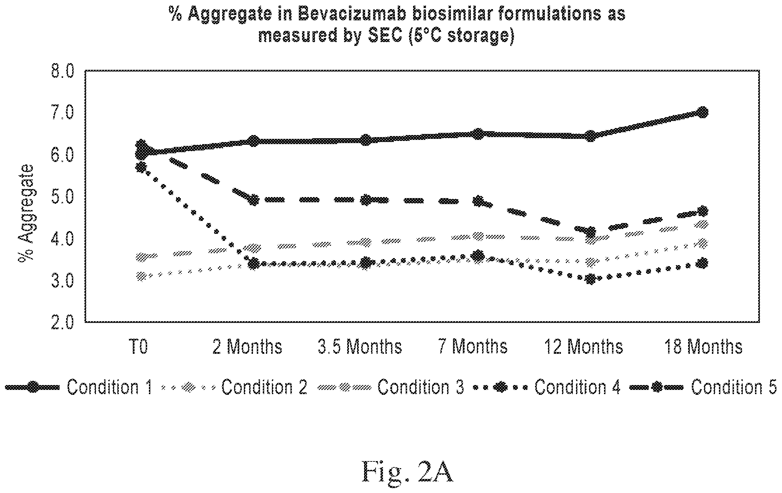

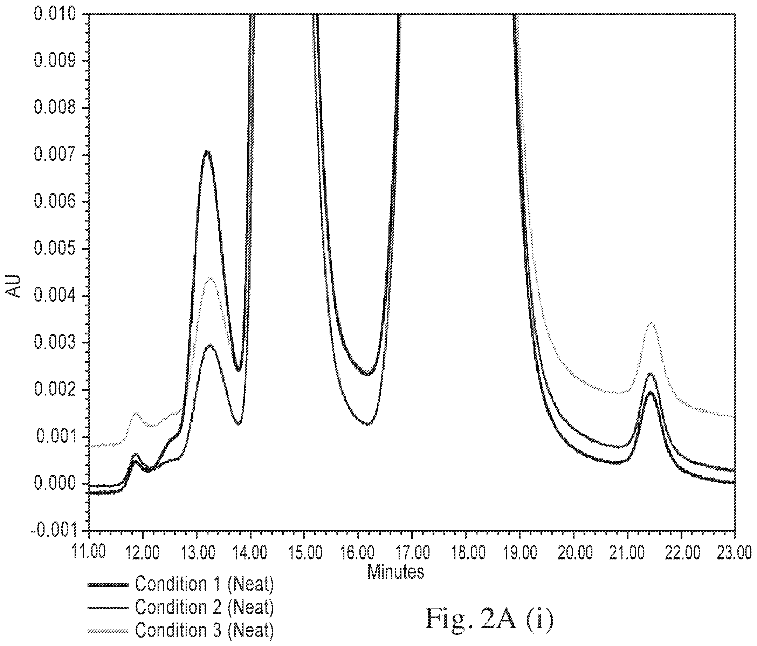

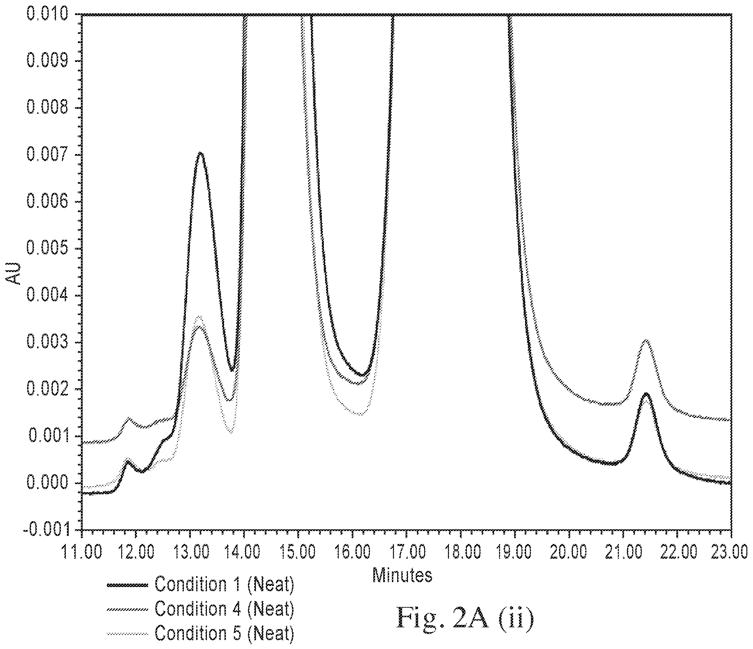

Figure 2A (i): shows the chromatographic overlay (as measured by size exclusion chromatography (SEC) using neat injection conditions and quantifies total aggregates) for Condition 1 (Bevacizumab (Avastin®) Match), Condition 2 (Bevacizumab Citrate Phosphate, pH 5.8) and Condition 3 (Bevacizumab Citrate Phosphate, pH 6.0) when product is stored at 5 °C over a duration of 18 months.
Figure 2A (ii) shows the chromatographic overlay (as measured by size exclusion chromatography (SEC) using neat injection conditions and quantifies total aggregates) for Condition 1 (Bevacizumab (Avastin®) Match), Condition 4 (Bevacizumab Acetate, pH 5.6), and Condition 5 (Bevacizumab Acetate, pH 5.8) when product is stored at 5 °C over a duration of 18 months.

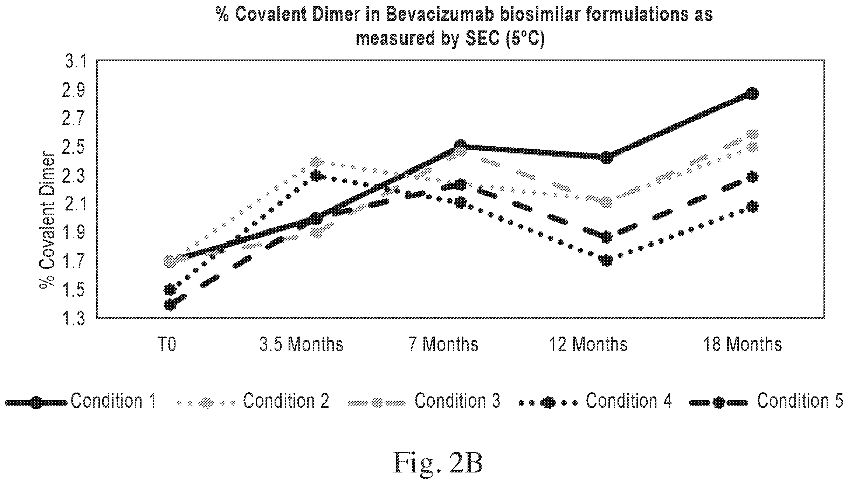

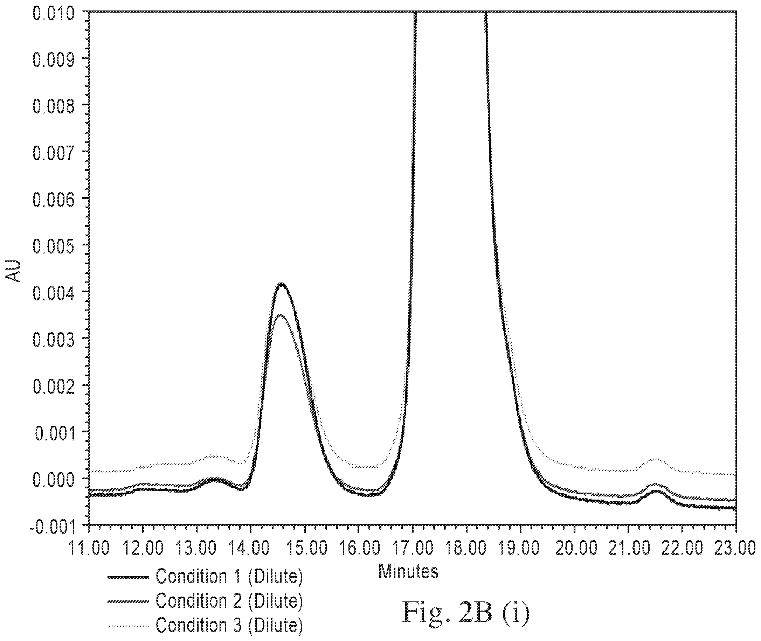

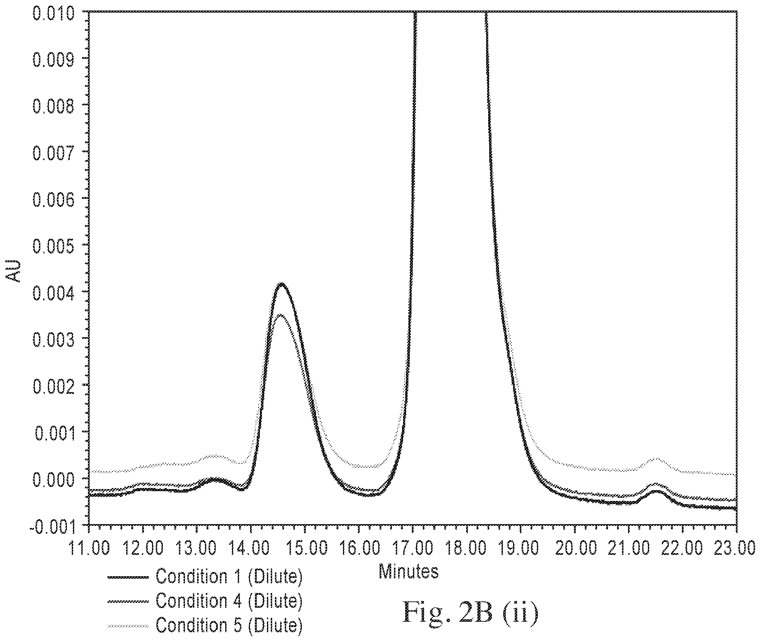

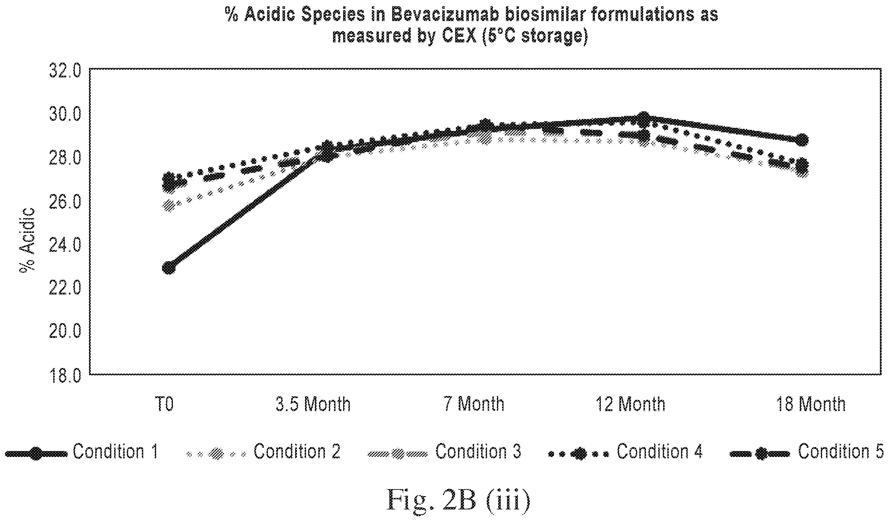

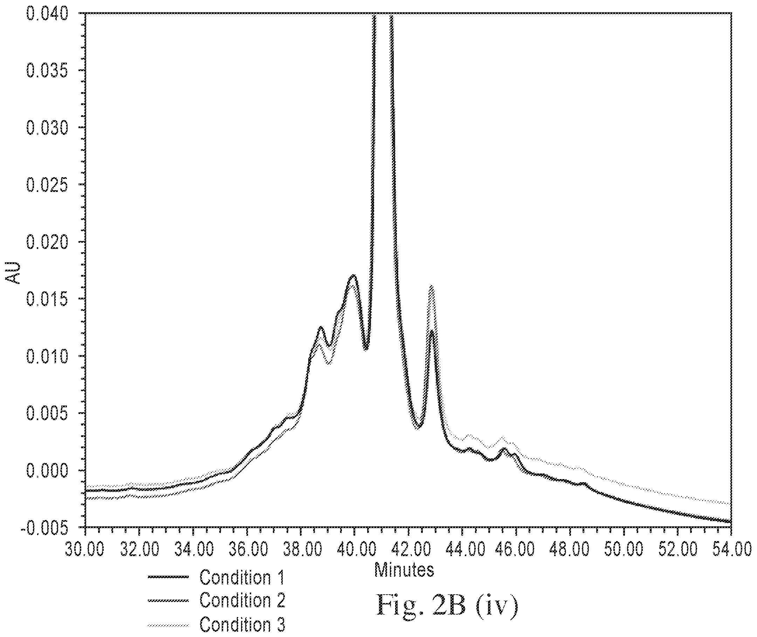

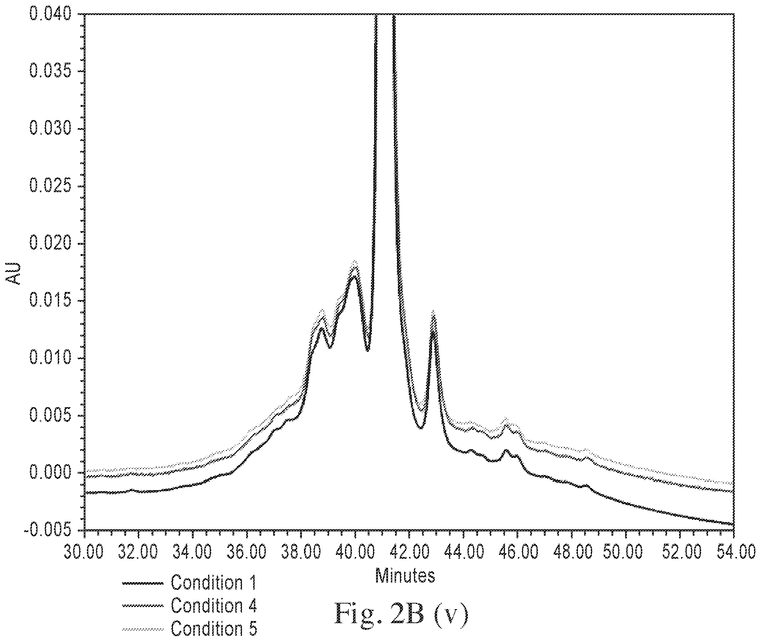

Figure 2B shows the percent of bevacizumab covalent dimer in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when stored at 5 °C over a duration of 18 months.
Figure 2B (i) shows the chromatographic overlay (as measured by size exclusion chromatography (SEC) using dilute injection conditions and quantifies bevacizumab covalent dimer) for Condition 1 (Bevacizumab (Avastin®) Match), Condition 2 (Bevacizumab Citrate Phosphate, pH 5.8) and Condition 3 (Bevacizumab Citrate Phosphate, pH 6.0) when product is stored at 5 °C over 18 months.
Figure 2B (ii) shows the chromatographic overlay (as measured by Size Exclusion Chromatography (SEC) using dilute injection conditions and quantifies the bevacizumab covalent dimer) for Condition 1 (Bevacizumab (Avastin®) Match), Condition 4 (Bevacizumab Acetate, pH 5.6), and Condition 5 (Bevacizumab Acetate, pH 5.8) when product is stored at 5 °C over 18 months.
Figure 2B (iii) shows the changes in percent of bevacizumab acidic species as measured by cation exchange chromatography (CEX) in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when stored at 5 °C over 18 months.
Figure 2B (iv) shows the chromatographic overlay (as measured by cation exchange chromatography (CEX) and quantifies % acidic, % basic and % main species) for Condition 1 (Bevacizumab (Avastin®) Match), Condition 2 (Bevacizumab Citrate Phosphate, pH 5.8) and Condition 3 (Bevacizumab Citrate Phosphate, pH 6.0) when product is stored at 5 °C over 18 months.
Figure 2B (v) shows the chromatographic overlay (as measured by cation exchange chromatography (CEX) and quantifies % acidic, % basic and % main species) for Condition 1 (Bevacizumab (Avastin®) Match), Condition 4 (Bevacizumab Acetate, pH 5.6), and Condition 5 (Bevacizumab Acetate, pH 5.8) when product is stored at 5 °C over 18 months.

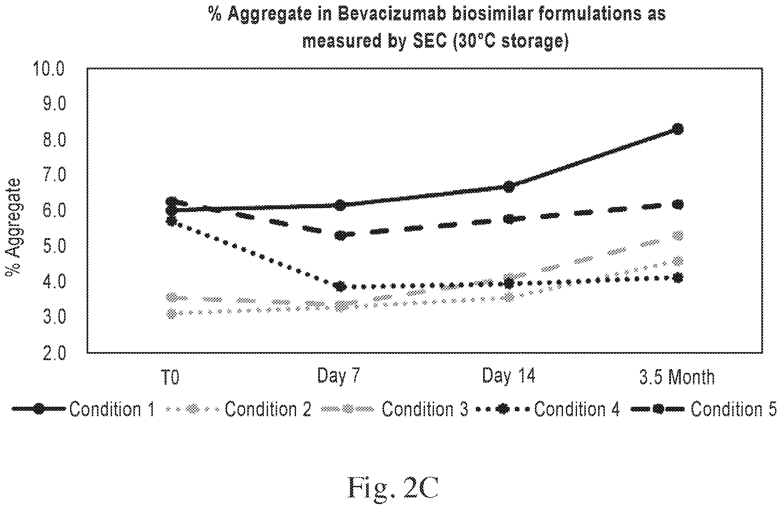

Figure 2C shows the percent of bevacizumab aggregates in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when stored at 30 °C.

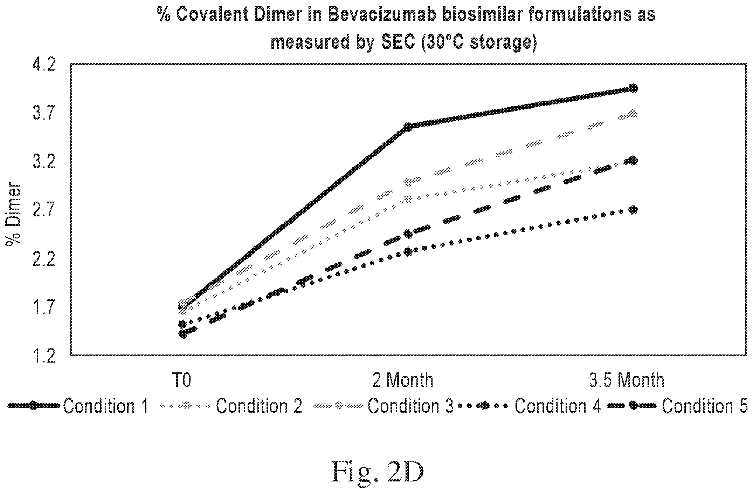

Figure 2D shows the percent of bevacizumab covalent dimer in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when stored at 30 °C.

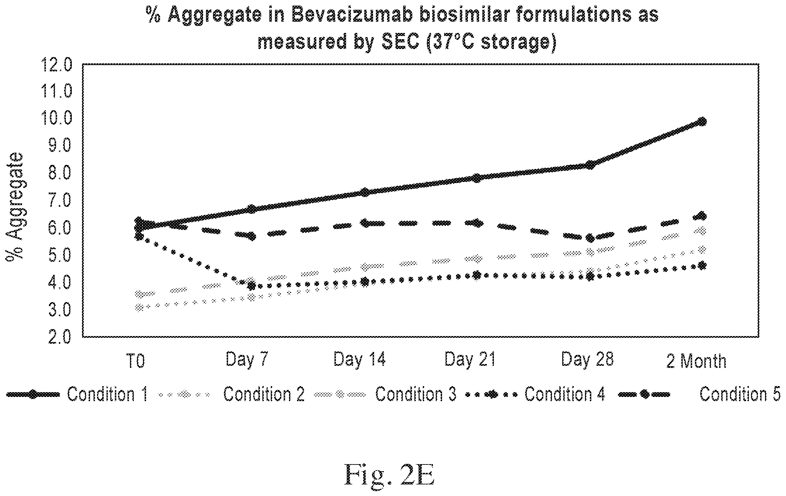

Figure 2E shows the percent of bevacizumab aggregates in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when stored at 37 °C.

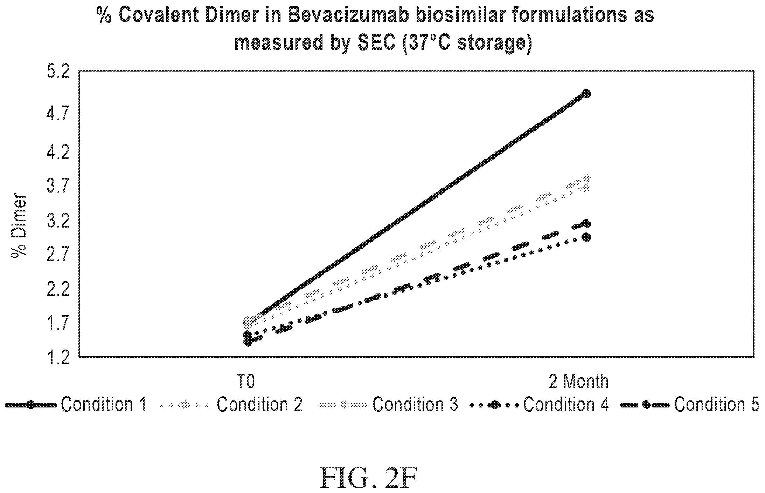

Figure 2F shows the percent of bevacizumab covalent dimer in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when stored at 37 °C.

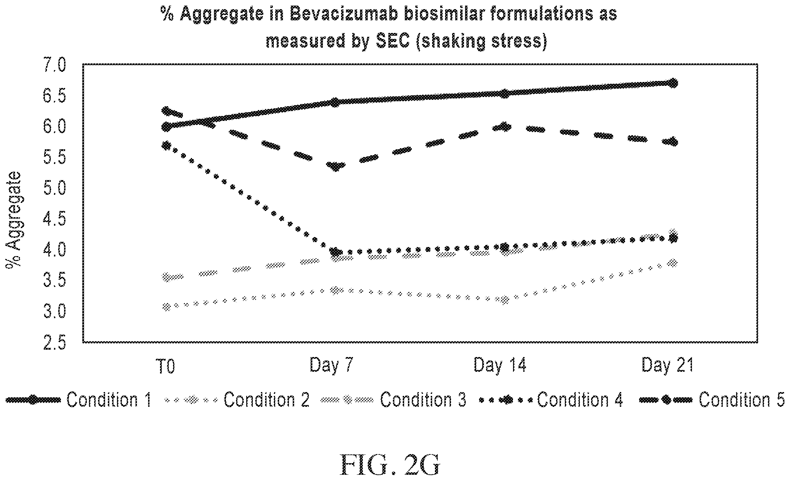

Figure 2G shows the percent of bevacizumab aggregates in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when subject to shaking stress.

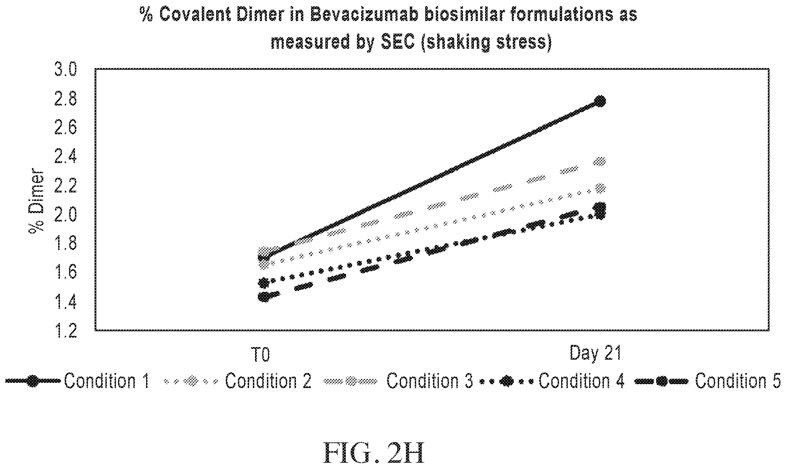

Figure 2H shows the percent of bevacizumab covalent dimer in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when subject to shaking stress.

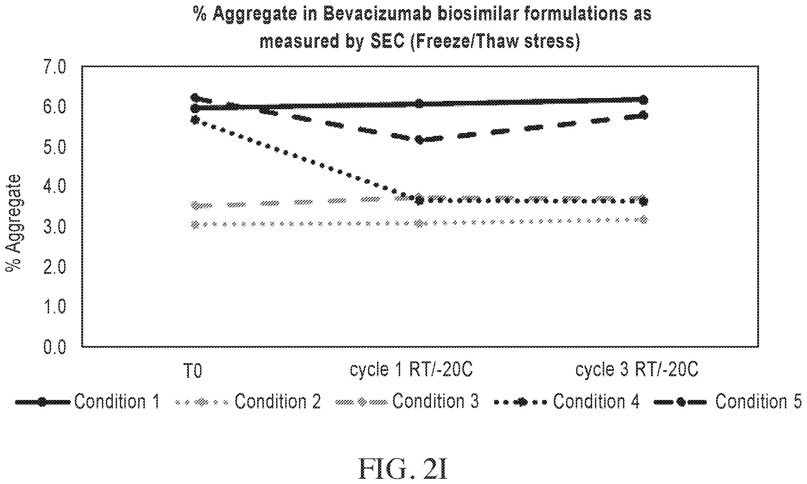

Figure 2I shows the percent of bevacizumab aggregates in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when subject to freeze/thaw stress.

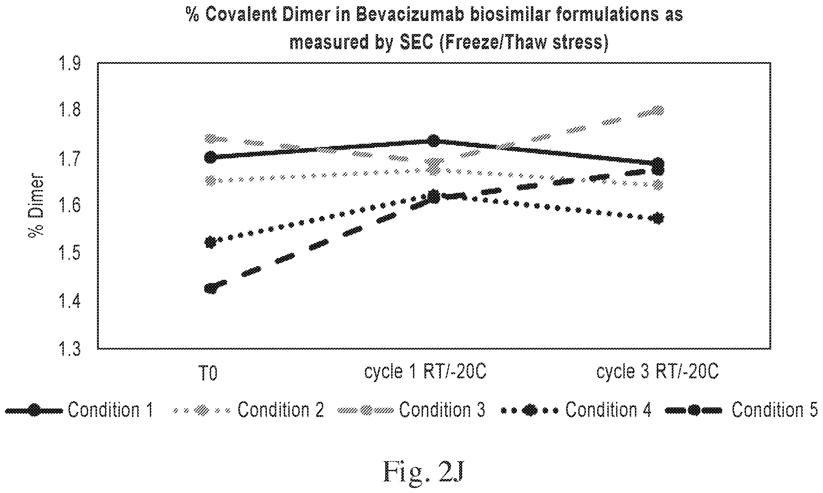

Figure 2J shows the percent of bevacizumab covalent dimer in Condition 1 (Bevacizumab (Avastin®) Match), Condition 2 (Bevacizumab Citrate Phosphate, pH 5.8), Condition 3 (Bevacizumab Citrate Phosphate, pH 6.0), Condition 4 (Bevacizumab Acetate, pH 5.6), and Condition 5 (Bevacizumab Acetate, pH 5.8), when subject to freeze/thaw stress.

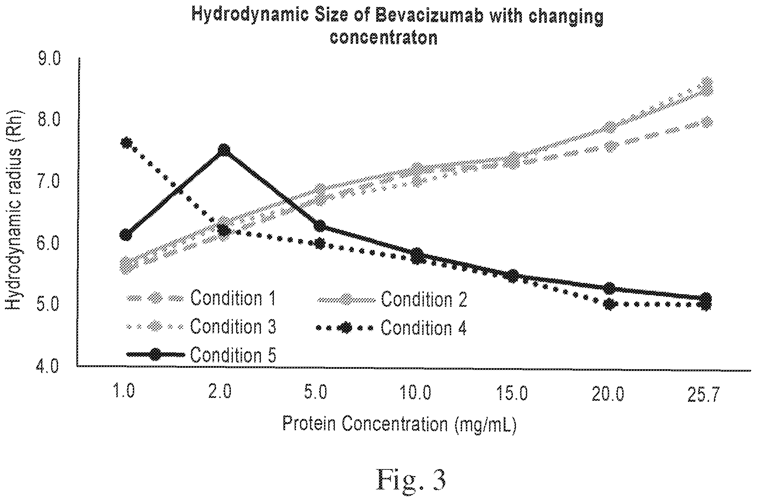

Figure 3 shows the hydrodynamic size of bevacizumab with changing concentration.

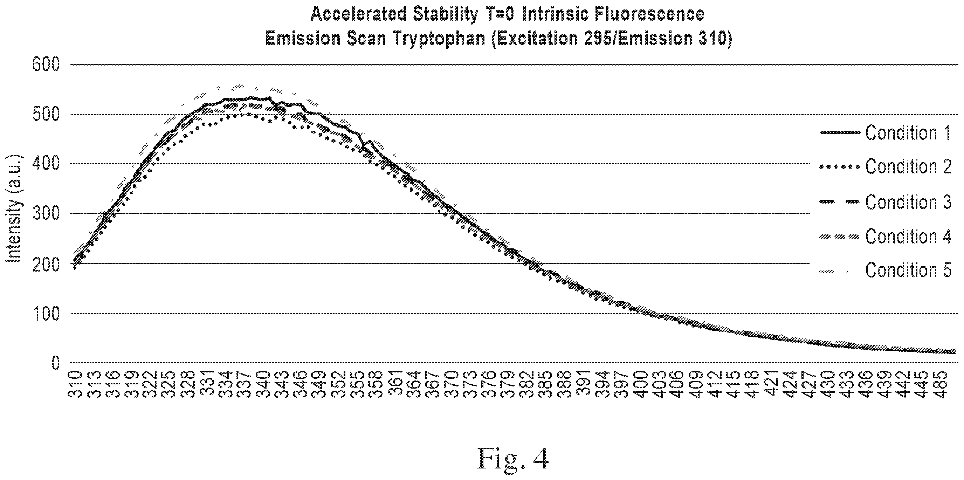

Figure 4 shows an accelerated stability T=0 Intrinsic Fluorescence Emission Scan Tryptophan plot.

### DETAILED DESCRIPTION OF THE INVENTION

Various terms relating to aspects of the present invention are used throughout the specification and claims. Such terms are to be given their ordinary meaning in the art, unless otherwise indicated. Other specifically defined terms are to be construed in a manner consistent with the definition provided herein.

As used herein, the singular forms "a," "an," and "the" include plural referents unless expressly stated otherwise.

As used herein, the terms "comprising," "having," and "including" encompass the more restrictive terms "consisting essentially of' and "consisting of."

The terms subject and patient are used interchangeably, and include any animal. Subjects include mammals, including companion and farm mammals, as well as rodents, including mice, rabbits, and rats, and other rodents. Non-human primates preferred subjects. Human beings are highly preferred subjects.

The terms composition and formulation are used interchangeably. Accordingly, a formulation of the disclosure may be a composition of the disclosure and a composition of the disclosure may be a formulation of the disclosure.

It has been observed in accordance with the present disclosure that formulations of a bevacizumab biosimilar antibody, which specifically binds to vascular endothelial growth factor, can be buffered with citrate phosphate, along with trehalose or sucrose, or buffered with acetate (instead of citrate phosphate) along with sucrose, with the buffers enhancing the thermal and colloidal stability of the antibody, even more so than formulations of bevacizumab (sold under the trade name Avastin®) currently approved for patient use. In particular, the inventive formulations demonstrated significantly lower antibody aggregation. The buffers enhance the shelf life of the antibody molecule. Accordingly, the disclosure features buffered formulations of a bevacizumab biosimilar antibody that include an aqueous carrier comprising a buffer comprising citrate phosphate, as well as trehalose or sucrose, at an acidic pH or, in the alternative, an aqueous carrier comprising a buffer comprising acetate, as well as sucrose, at an acidic pH.

The antibody specifically binds to an epitope on vascular endothelial growth factor (VEGF), and the epitope may be linear or conformational. In some preferred aspects, the antibody comprises a heavy chain comprising the amino acid sequence of SEQ ID NO: 1. In some preferred aspects, the antibody comprises a light chain comprising the amino acid sequence of SEQ ID NO: 2. Preferably, the antibody comprises a heavy chain constant domain and/or a light chain constant domain. In highly preferred aspects, the antibody comprises a heavy chain comprising the amino acid sequence of SEQ ID NO: 1 and a light chain comprising the amino acid sequence of SEQ ID NO: 2. In some aspects, the antibody comprises a heavy chain variable region of the amino acid sequence of SEQ ID NO: 3 and the light chain variable region of the amino acid sequence of SEQ ID NO:4.

| **Bevacizumab Heavy Chain IgG1 (SEQ ID NO:1)** |
|---|
| |

| **Bevacizumab Light Chain (SEQ ID NO: 2)** |
|---|
| |

| **Bevacizumab Heavy Chain Variable Region (SEQ ID NO: 3)** |
|---|
| |

| **Bevacizumab Light Chain Variable Region (SEQ ID NO: 4)** |
|---|
| |

Preferably, the antibody is a full-length antibody, comprising both variable and constant regions, although in some aspects, the antibody may comprise a derivative or fragment or portion of a full-length antibody that retains the antigen-binding specificity, and also preferably retains most or all of the affinity, of the full length antibody molecule. The antibody may comprise post-translational modifications (PTMs) or moieties, which may impact antibody activity or stability. The antibody may be methylated, acetylated, glycosylated, sulfated, phosphorylated, carboxylated, and/or amidated, and may comprise other moieties that are well known in the art.

The formulation preferably comprises a therapeutically effective amount of the antibody. A therapeutically effective amount may vary, depending on the disease or condition being treated upon administration of the antibody, and/or depending on the characteristics of the subject to which the antibody is administered, such as age, gender, height, weight, state of advancement or stage of the disease or condition, the number and efficacy of previous administrations, other therapeutic agents administered to the subject, and other characteristics that are known to the practitioner or that would otherwise be taken into account in determining appropriate dosing. Preferably, a therapeutically effective amount is an amount that is effective to treat cancers such as non-squamous non-small cell lung cancer, glioblastoma, renal cell carcinoma, cervical cancer, or epithelial ovarian, fallopian tube, or primary peritoneal cancer.

The formulation may comprise from about 10 mg/ml to about 50 mg/ml of the antibody. In some aspects, the formulation comprises from about 10 mg/ml to about 40 mg/ml of the antibody. In some aspects, the formulation comprises from about 10 mg/ml to about 30 mg/ml of the antibody. In some aspects, the formulation comprises from about 20 mg/ml to about 50 mg/ml of the antibody. In some aspects, the formulation comprises from about 20 mg/ml to about 40 mg/ml of the antibody. In some aspects, the formulation comprises from about 20 mg/ml to about 30 mg/ml of the antibody. In some aspects, the formulation comprises from about 15 mg/ml to about 45 mg/ml of the antibody. In some aspects, the formulation comprises from about 15 mg/ml to about 35 mg/ml of the antibody. In some aspects, the formulation comprises from about 15 mg/ml to about 30 mg/ml of the antibody. In some aspects, the formulation comprises from about 21 mg/ml to about 29 mg/ml of the antibody. In some aspects, the formulation comprises from about 22 mg/ml to about 28 mg/ml of the antibody. In some aspects, the formulation comprises from about 23 mg/ml to about 27 mg/ml of the antibody. In some aspects, the formulation comprises from about 24 mg/ml to about 25 mg/ml of the antibody. In some aspects, the formulation comprises from about 25 mg/ml to about 30 mg/ml of the antibody. In some aspects, the formulation comprises from about 25 mg/ml to about 26 mg/ml of the antibody. In some aspects, the formulation comprises from about 25 mg/ml to about 27 mg/ml of the antibody. In some aspects, the formulation comprises from about 25 mg/ml to about 28 mg/ml of the antibody. In some aspects, the formulation comprises from about 25 mg/ml to about 29 mg/ml of the antibody. In some aspects, the formulation comprises from about 25 mg/ml to about 30 mg/ml of the antibody. In some aspects, the formulation comprises from about 24 mg/ml to about 27 mg/ml of the antibody. In some aspects, the formulation comprises from about 24 mg/ml to about 28 mg/ml of the antibody. In some aspects, the formulation comprises from about 24 mg/ml to about 29 mg/ml of the antibody. In some aspects, the formulation comprises from about 24 mg/ml to about 30 mg/ml of the antibody. In some aspects, the formulation comprises from about 25.5 mg/ml to about 26 mg/ml of the antibody. In some aspects, the formulation comprises from about 25.4 mg/ml to about 25.9 mg/ml of the antibody. In some aspects, the formulation comprises from about 25.6 mg/ml to about 25.9 mg/ml of the antibody. In some aspects, the formulation comprises from about 25.5 mg/ml to about 25.8 mg/ml of the antibody. In some aspects, the formulation comprises from about 25.5 mg/ml to about 25.7 mg/ml of the antibody. These ranges include the lower and upper amounts that define the range. In some aspects, the formulation comprises about 25 mg/ml of the antibody. In some aspects, the formulation comprises about 25.5 mg/ml of the antibody. In some aspects, the formulation comprises about 25.6 mg/ml of the antibody. In some aspects, the formulation comprises about 25.7 mg/ml of the antibody. In some aspects, the formulation comprises about 25.8 mg/ml of the antibody.

The antibody, for example, at the concentrations described or exemplified herein, is preferably formulated with a buffered aqueous carrier, and the carrier preferably comprises water. The buffered antibody formulation is preferably in liquid form, and more preferably in liquid form suitable for intravenous administration. Thus, the amount of water in the buffered formulation may vary in accordance with the desired volume of the infusion. In some preferred aspects, the buffer comprises citrate phosphate, trehalose, and a mild surfactant such as polysorbate 20, and maintains the antibody formulation at an acidic pH of from about 5.8 to about 6.0. In some alternate preferred aspects, the buffer comprises acetate, sucrose, and a mild surfactant such as polysorbate 20, and maintains the antibody formulation at an acidic pH of from about 5.6 to about 5.8. When stored in the buffered formulation, the antibody is shelf-stable under normal storage conditions.

Citrate phosphate comprises an aqueous combination of dibasic sodium phosphate dodecahydrate and citric acid monohydrate, in a pre-mixed solution comprising about 0.2 M of dibasic sodium phosphate and about 0.1 M of citric acid.

The buffer may comprise from about 10 mM to about 100 mM of citrate phosphate. In some aspects, the buffer may comprise from about 20 mM to about 90 mM of citrate phosphate. In some aspects, the buffer may comprise from about 30 mM to about 70 mM of citrate phosphate. In some aspects, the buffer may comprise from about 30 mM to about 80 mM of citrate phosphate. In some aspects, the buffer may comprise from about 40 mM to about 70 mM of citrate phosphate. In some aspects, the buffer may comprise from about 40 mM to about 60 mM of citrate phosphate. In some aspects, the buffer may comprise from about 45 mM to about 55 mM of citrate phosphate. In some aspects, the buffer may comprise from about 46 mM to about 54 mM of citrate phosphate rate. In some aspects, the buffer may comprise from about 47 mM to about 53 mM of citrate phosphate. In some aspects, the buffer may comprise from about 48 mM to about 52 mM of citrate phosphate. In some aspects, the buffer may comprise from about 49 mM to about 51 mM of citrate phosphate. In some aspects, the buffer may comprise from about 40 mM to about 50 mM of citrate phosphate. In some aspects, the buffer may comprise from about 50 mM to about 75 mM of citrate phosphate. In some aspects, the buffer may comprise from about 30 mM to about 55 mM of citrate phosphate. In some aspects, the buffer may comprise from about 40 mM to about 55 mM of citrate phosphate. In some aspects, the buffer may comprise from about 42 mM to about 52 mM of citrate phosphate. In some aspects, the buffer may comprise from about 46 mM to about 52 mM of citrate phosphate. In some aspects, the buffer may comprise from about 43 mM to about 53 mM of citrate phosphate. These ranges include the lower and upper amounts that define the range. In some aspects, the buffer comprises about 50 mM of citrate phosphate.

The citrate phosphate buffer may comprise from about 100 mM to about 200 mM of trehalose. In some aspects, the buffer may comprise from about 110 mM to about 190 mM of trehalose. In some aspects, the buffer may comprise from about 120 mM to about 180 mM of trehalose. In some aspects, the buffer may comprise from about 130 mM to about 170 mM of trehalose. In some aspects, the buffer may comprise from about 140 mM to about 170 mM of trehalose. In some aspects, the buffer may comprise from about 150 mM to about 170 mM of trehalose. In some aspects, the buffer may comprise from about 155 mM to about 165 mM of trehalose. In some aspects, the buffer may comprise from about 150 mM to about 160 mM of trehalose. In some aspects, the buffer may comprise from about 153 mM to about 164 mM of trehalose. In some aspects, the buffer may comprise from about 152 mM to about 167 mM of trehalose. In some aspects, the buffer may comprise from about 154 mM to about 164 mM of trehalose. In some aspects, the buffer may comprise from about 155 mM to about 163 mM of trehalose. In some aspects, the buffer may comprise from about 156 mM to about 162 mM of trehalose. In some aspects, the buffer may comprise from about 157 mM to about 161 mM of trehalose. In some aspects, the buffer may comprise from about 158 mM to about 160 mM of trehalose. In some aspects, the buffer may comprise from about 158.5 mM to about 158.9 mM of trehalose. In some aspects, the buffer may comprise from about 158.6 mM to about 158.8 mM of trehalose. In some aspects, the buffer may comprise from about 158 mM to about 161 mM of trehalose. In some aspects, the buffer may comprise from about 159 mM to about 161 mM of trehalose. In some aspects, the buffer may comprise from about 157 mM to about 160 mM of trehalose. In some aspects, the buffer may comprise from about 157 mM to about 159 mM of trehalose. In some aspects, the buffer may comprise from about 150 mM to about 159 mM of trehalose. In some aspects, the buffer may comprise from about 159 mM to about 160 mM of trehalose. In some aspects, the buffer may comprise from about 159 mM to about 165 mM of trehalose. These ranges include the lower and upper amounts that define the range. In some aspects, the buffer comprises about 159 mM of trehalose. In some aspects, the buffer comprises about 158.7 mM of trehalose. In some aspects, sucrose may be used in any of these concentrations in place of trehalose. Thus, for example, the citrate phosphate buffer may comprise sucrose as a stabilizer instead of trehalose.

The acetate-sucrose buffer may comprise from about 1 mM to about 30 mM of acetate. In some aspects, the buffer may comprise from about 5 mM to about 25 mM of acetate. In some aspects, the buffer may comprise from about 10 mM to about 20 mM of acetate. In some aspects, the buffer may comprise from about 11 mM to about 19 mM of acetate. In some aspects, the buffer may comprise from about 12 mM to about 18 mM of acetate. In some aspects, the buffer may comprise from about 13 mM to about 15 mM of acetate. In some aspects, the buffer may comprise from about 10 mM to about 15 mM of acetate. In some aspects, the buffer may comprise from about 12 mM to about 16 mM of acetate. In some aspects, the buffer may comprise from about 12 mM to about 15 mM of acetate. In some aspects, the buffer may comprise from about 13 mM to about 16 mM of acetate. In some aspects, the buffer may comprise from about 13 mM to about 17 mM of acetate. In some aspects, the buffer may comprise from about 14 mM to about 18 mM of acetate. In some aspects, the buffer may comprise from about 14 mM to about 16 mM of acetate. In some aspects, the buffer may comprise from about 15 mM to about 20 mM of acetate. In some aspects, the buffer may comprise from about 5 mM to about 15 mM of acetate. In some aspects, the buffer may comprise from about 11 mM to about 17 mM of acetate. In some aspects, the buffer may comprise from about 15 mM to about 16 mM of acetate. These ranges include the lower and upper amounts that define the range. In some aspects, the buffer comprises about 15 mM of acetate. Preferably, the acetate is sodium acetate trihydrate.

The acetate-sucrose or citrate phosphate-sucrose buffer may comprise from about 100 mM to about 250 mM of sucrose. In some aspects, the buffer may comprise from about 125 mM to about 225 mM of sucrose. In some aspects, the buffer may comprise from about 150 mM to about 200 mM of sucrose. In some aspects, the buffer may comprise from about 155 mM to about 195 mM of sucrose. In some aspects, the buffer may comprise from about 160 mM to about 190 mM of sucrose. In some aspects, the buffer may comprise from about 165 mM to about 185 mM of sucrose. In some aspects, the buffer may comprise from about 166 mM to about 184 mM of sucrose. In some aspects, the buffer may comprise from about 167 mM to about 183 mM of sucrose. In some aspects, the buffer may comprise from about 168 mM to about 182 mM of sucrose. In some aspects, the buffer may comprise from about 169 mM to about 181 mM of sucrose. In some aspects, the buffer may comprise from about 170 mM to about 180 mM of sucrose. In some aspects, the buffer may comprise from about 171 mM to about 179 mM of sucrose. In some aspects, the buffer may comprise from about 172 mM to about 178 mM of sucrose. In some aspects, the buffer may comprise from about 174 mM to about 177 mM of sucrose. In some aspects, the buffer may comprise from about 174 mM to about 176 mM of sucrose. In some aspects, the buffer may comprise from about 175 mM to about 175.5 mM of sucrose. In some aspects, the buffer may comprise from about 175.2 mM to about 175.4 mM of sucrose. In some aspects, the buffer may comprise from about 175 mM to about 185 mM of sucrose. In some aspects, the buffer may comprise from about 165 mM to about 175 mM of sucrose. In some aspects, the buffer may comprise from about 170 mM to about 190 mM of sucrose. In some aspects, the buffer may comprise from about 150 mM to about 175 mM of sucrose. These ranges include the lower and upper amounts that define the range. In some aspects, the buffer comprises about 175 mM of sucrose. In some aspects, the buffer comprises about 175.3 mM of sucrose.

The antibody formulation (e.g., with the citrate phosphate-trehalose or the acetate sucrose buffer) preferably comprises a non-ionic surfactant. More preferably, the non-ionic surfactant comprises polysorbate 20 (may comprise Tween® 20 brand polysorbate of Croda International Plc, Yorkshire, England). The antibody formulation, including the antibody and the aqueous buffer, preferably comprises from about 0.01% to about 0.1% (by volume) of polysorbate 20. In some aspects, the antibody formulation comprises from about 0.02% to about 0.09% (by volume) of polysorbate 20. In some aspects, the antibody formulation comprises from about 0.03% to about 0.08% (by volume) of polysorbate 20. In some aspects, the antibody formulation comprises from about 0.01% to about 0.07% (by volume) of polysorbate 20. In some aspects, the antibody formulation comprises from about 0.02% to about 0.06% (by volume) of polysorbate 20. In some aspects, the antibody formulation comprises from about 0.03% to about 0.05% (by volume) of polysorbate 20. In some aspects, the antibody formulation comprises from about 0.04% to about 0.06% (by volume) polysorbate 20. In some aspects, the antibody formulation comprises from about 0.02% to about 0.05% (by volume) of polysorbate 20. In some aspects, the antibody formulation comprises from about 0.02% to about 0.04% (by volume) of polysorbate 20. In some aspects, the antibody formulation comprises from about 0.03% to about 0.06% (by volume) of polysorbate 20. In some aspects, the antibody formulation comprises from about 0.01% to about 0.05% (by volume) of polysorbate 20. In some aspects, the antibody formulation comprises from about 0.03% to about 0.04% (by volume) of polysorbate 20. In some aspects, the antibody formulation comprises from about 0.04% to about 0.05% (by volume) of polysorbate 20. In some aspects, the antibody formulation comprises from about 0.035% to about 0.045% (by volume) of polysorbate 20. These ranges include the lower and upper amounts that define the range. In some aspects, the antibody formulation comprises about 0.04% (by volume) of polysorbate 20.

The antibody formulation (e.g., with the citrate phosphate-trehalose/sucrose or the acetate sucrose buffer) preferably is buffered to an acidic pH. The formulation preferably has a pH of from about 5.3 to about 6.5. In some aspects, the formulation has a pH of about 5.4 to about 6.4. In some preferred aspects, the formulation has a pH of about 5.4 to about 5.9. In some preferred aspects, the formulation has a pH of about 5.5 to about 5.8. In some preferred aspects, the formulation has a pH of about 5.6 to about 5.8. In some preferred aspects, the formulation has a pH of about 5.6 to about 5.9. In some aspects, the formulation has a pH of about 5.5 to about 5.3. In some preferred aspects, the formulation has a pH of about 5.6 to about 6.2. In some aspects, the formulation has a pH of about 5.7 to about 6.1. In some aspects, the formulation has a pH of about 5.8 to about 6.0. In some preferred aspects, the formulation has a pH of about 5.4 to about 5.9. In some aspects, the formulation has a pH of about 5.6 to about 5.9. In some preferred aspects, the formulation has a pH of about 5.7 to about 5.9. In some preferred aspects, the formulation has a pH of about 5.9 to about 6.1. In some aspects, the formulation has a pH of about 6.0 to about 6.2. In some aspects, the formulation has a pH of about 5.7 to about 6.0. In some preferred aspects, the formulation has a pH of from about 5.8 to about 6.1. These ranges include the lower and upper amounts that define the range. In some aspects, the formulation has a pH of about 5.8. In some aspects, the formulation has a pH of about 5.9. In some aspects, the formulation has a pH of about 6.0.

In some preferred aspects, the antibody formulation comprises from about 20 mg/ml to about 30 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 30 mM to about 70 mM of citrate phosphate, from about 150 mM to about 170 mM of trehalose, and from about 0.01% to about 0.07% (by volume) of polysorbate 20, and has a pH of from about 5.6 to about 6.0. In some aspects, the antibody formulation consists essentially of from about 20 mg/ml to about 30 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 30 mM to about 70 mM of citrate phosphate, from about 150 mM to about 170 mM of trehalose, and from about 0.01% to about 0.07% (by volume) of polysorbate 20, and has a pH of from about 5.6 to about 6.0. In some aspects, the antibody formulation consists of from about 20 mg/ml to about 30 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 30 mM to about 70 mM of citrate phosphate, from about 150 mM to about 170 mM of trehalose, and from about 0.01% to about 0.07% (by volume) of polysorbate 20, and has a pH of from about 5.6 to about 6.0. In any such embodiments, the antibody may be present in the formulation at from about 21 mg/ml to about 29 mg/ml, or from about 22 mg/ml to about 28 mg/ml, or from about 23 mg/ml to about 27 mg/ml, of from about 24 mg/ml to about 26 mg/ml, or from about 24.5 mg/ml to about 26.5 mg/ml, about 25 mg/ml, about 26 mg/ml, about 25.5 mg/ml, about 25.6 mg/ml, about 25.7 mg/ml or about 25.8 mg/ml.

In some preferred aspects, the antibody formulation comprises from about 20 mg/ml to about 30 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 40 mM to about 60 mM of citrate phosphate, from about 154 mM to about 164 mM of trehalose, and from about 0.02% to about 0.06% (by volume) of polysorbate 20, and has a pH of from about 5.6 to about 6.0, or a pH of about 5.8, or a pH of about 6.0. In some aspects, the antibody formulation consists essentially of from about 20 mg/ml to about 30 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 40 mM to about 60 mM of citrate phosphate, from about 154 mM to about 164 mM of trehalose, and from about 0.02% to about 0.06% (by volume) of polysorbate 20, and has a pH of from about 5.6 to about 6.0, or a pH of about 5.8, or a pH of about 6.0. In some aspects, the antibody formulation consists of from about 20 mg/ml to about 30 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 40 mM to about 60 mM of citrate phosphate, from about 154 mM to about 164 mM of trehalose, and from about 0.02% to about 0.06% (by volume) of polysorbate 20, and has a pH of from about 5.6 to about 6.0, or a pH of about 5.8, or a pH of about 6.0. In any such embodiments, the antibody may be present in the formulation at from about 21 mg/ml to about 29 mg/ml, or from about 22 mg/ml to about 28 mg/ml, or from about 23 mg/ml to about 27 mg/ml, of from about 24 mg/ml to about 26 mg/ml, or from about 24.5 mg/ml to about 26.5 mg/ml, about 25 mg/ml, about 26 mg/ml, about 25.5 mg/ml, about 25.6 mg/ml, about 25.7 mg/ml or about 25.8 mg/ml.

In some preferred aspects, the antibody formulation comprises from about 25 mg/ml to about 26.5 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 45 mM to about 55 mM of citrate phosphate, from about 157 mM to about 161 mM of trehalose, and from about 0.03% to about 0.05% (by volume) of polysorbate 20, and has a pH of from about 5.6 to about 6.0, or a pH of about 5.8, or a pH of about 6.0. In some aspects, the antibody formulation consists essentially of from about 25 mg/ml to about 26.5 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 45 mM to about 55 mM of citrate phosphate, from about 157 mM to about 161 mM of trehalose, and from about 0.03% to about 0.05% (by volume) of polysorbate 20, and has a pH of from about 5.6 to about 6.0, or a pH of about 5.8, or a pH of about 6.0. In some aspects, the antibody formulation consists of from about 25 mg/ml to about 26.5 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising from about 45 mM to about 55 mM of citrate phosphate, from about 157 mM to about 161 mM of trehalose, and from about 0.03% to about 0.05% (by volume) of polysorbate 20, and has a pH of from about 5.6 to about 6.0, or a pH of about 5.8, or a pH of about 6.0. In any such embodiments, the antibody may be present in the formulation at from about 25 mg/ml to about 26 mg/ml, or from about 25.5 mg/ml to about 26 mg/ml, about 25 mg/ml, about 26 mg/ml, about 25.5 mg/ml, about 25.6 mg/ml, about 25.7 mg/ml or about 25.8 mg/ml.

In some preferred aspects, the antibody formulation comprises from about 25.5 mg/ml to about 26.1 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising about 50 mM of citrate phosphate, about 159 mM of trehalose, and about 0.04% (by volume) of polysorbate 20, and has a pH of about 5.8 or about 6.0. In some aspects, the antibody formulation consists essentially of from about 25.5 mg/ml to about 26.1 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising about 50 mM of citrate phosphate, about 159 mM of trehalose, and about 0.04% (by volume) of polysorbate 20, and has a pH of about 5.8 or about 6.0. In some aspects, the antibody formulation consists of from about 25.5 mg/ml to about 26.1 mg/ml of an antibody that specifically binds to VEGF and comprises a heavy chain comprising the amino acid sequence of SEQ ID NO: 1 and a light chain comprising the amino acid sequence of SEQ ID NO: 2, a buffer comprising about 50 mM of citrate phosphate, about 159 mM of trehalose, and about 0.04% (by volume) of polysorbate 20, and has a pH of about 5.8 or about 6.0. In any such embodiments, the antibody may be present in the formulation at about 26 mg/ml, about 25.5 mg/ml, about 25.6 mg/ml, about 25.7 mg/ml or about 25.8 mg/ml.

The formulation stabilizes the antibody for improved shelf storage, particularly over a period of months to years. When stored in the formulation, the antibody maintains thermal and colloidal stability during the period of storage. For example, when stored in the formulation, the antibody is stable and exhibits minimal aggregation, flocculation, fragmentation, and denaturation, and the antibody retains its VEGF binding activity.

It is preferred that the antibody formulation be stored under refrigerated conditions, and preferably at a temperature of from about 2 °C to about 6 °C, including about 2 °C, about 3 °C, about 4 °C, about 5 °C, about 6 °C, about 7 °C about 8 °C. The antibody formulation may be stored at such temperatures for at least about 3 months. In some aspects, the antibody formulation may be stored at such temperatures for at least about 6 months. In some aspects, the antibody formulation may be stored at such temperatures for at least about 9 months. In some aspects, the antibody formulation may be stored at such temperatures for at least about 12 months. In some aspects, the antibody formulation may be stored at such temperatures for at least about 15 months. In some aspects, the antibody formulation may be stored at such temperatures for at least about 18 months. In some aspects, the antibody formulation may be stored at such temperatures for at least about 21 months. In some aspects, the antibody formulation may be stored at such temperatures for at least about 24 months. During the storage period the antibody is stable and exhibits minimal aggregation, flocculation, fragmentation, and denaturation, and the antibody retains it VEGF binding activity such that the antibody formulation may be removed from storage, administered to a patient, and still exhibit therapeutic efficacy against the condition for which the formulation is administered.

The formulation preferably comprises about 20 mg/ml to about 30 mg/ml of antibody and, more preferably about 25 mg/ml or about 25.5 mg/ml, or about 26 mg/ml of antibody. Among this amount of antibody protein is a percentage of antibody monomers in active, native form, as well as a percentage of antibody aggregates that have reduced or no VEGF binding activity. It is highly preferred that that the formulation include a maximal amount of functional antibody monomers and a minimal amount of antibody aggregates, and structurally altered forms of the antibody with reduced binding activity and/or therapeutic efficacy (relative to the unaltered monomer). For example, the antibody formulation preferably contains at least about 85% by weight of antibody monomers, and less than about 15% by weight of antibody aggregates with reduced VEGF binding activity and/or therapeutic efficacy when stored at about 2 °C to about 6 °C for at least about six months.

In some aspects, the antibody formulation contains at least about 90% by weight of antibody monomers, and less than about 10% by weight of antibody aggregates with reduced VEGF binding activity and/or therapeutic efficacy when stored at about 2 °C to about 8 °C for at least about six months. In some aspects, the antibody formulation contains at least about 93% by weight of antibody monomers, and less than about 7% by weight of antibody aggregates with reduced VEGF binding activity and/or therapeutic efficacy when stored at about 2 °C to about 8 °C for at least about six months. In some aspects, the antibody formulation contains at least about 95% by weight of antibody monomers, and less than about 5% by weight of antibody aggregates with reduced VEGF binding activity and/or therapeutic efficacy when stored at about 2 °C to about 8 °C for at least about six months. In some aspects, the antibody formulation contains at least about 96% by weight of antibody aggregates with reduced VEGF binding activity and/or therapeutic efficacy when stored at about 2 °C to about 8 °C for at least about six months. In some aspects, the antibody formulation contains at least about 97% by weight of antibody monomers, and less than about 3% by weight of antibody aggregates with reduced VEGF binding activity and/or therapeutic efficacy when stored at about 2 °C to about 8 °C for at least about six months. In some aspects, the antibody formulation contains at least about 98% by weight of antibody monomers, and less than about 2% by weight of antibody aggregates with reduced VEGF binding activity and/or therapeutic efficacy when stored at about 2 °C to about 8 °C for at least about six months. In some aspects, the antibody formulation contains at least about 99% by weight of antibody monomers, and less than about 1% by weight of antibody aggregates with reduced VEGF binding activity and/or therapeutic efficacy when stored at about 2 °C to about 8 °C for at least about six months. The amount of antibody monomers and/or antibody aggregates may be determined according to any technique suitable in the art, including those described or exemplified herein, including any one or combination of differential light scattering (DLS), differential scanning calorimetry (DSC), size exclusion chromatography (SE-HPLC), non-reducing and reducing capillary electrophoresis SDS (NR CE-SDS and R CE-SDS), and particulate count (PC).

In some aspects, the antibody formulation contains at least about 90% by weight of antibody monomers, and less than about 10% by weight of antibody aggregates with reduced VEGF binding activity and/or therapeutic efficacy when stored at about 2 °C to about 8 °C for at least about twelve months. In some aspects, the antibody formulation contains at least about 93% by weight of antibody monomers, and less than about 7% by weight of antibody aggregates with reduced VEGF binding activity and/or therapeutic efficacy when stored at about 2 °C to about 8 °C for at least about twelve months. In some aspects, the antibody formulation contains at least about 95% by weight of antibody monomers, and less than about 5% by weight of antibody aggregates with reduced VEGF binding activity and/or therapeutic efficacy when stored at about 2 °C to about 8 °C for at least about twelve months. In some aspects, the antibody formulation contains at least about 96% by weight of antibody aggregates with reduced VEGF binding activity and/or therapeutic efficacy when stored at about 2 °C to about 8 °C for at least about twelve months. In some aspects, the antibody formulation contains at least about 97% by weight of antibody monomers, and less than about 3% by weight of antibody aggregates with reduced VEGF binding activity and/or therapeutic efficacy when stored at about 2 °C to about 8 °C for at least about twelve months. In some aspects, the antibody formulation contains at least about 98% by weight of antibody monomers, and less than about 2% by weight of antibody aggregates with reduced VEGF binding activity and/or therapeutic efficacy when stored at about 2 °C to about 8 °C for at least about twelve months. In some aspects, the antibody formulation contains at least about 99% by weight of antibody monomers, and less than about 1% by weight of antibody aggregates with reduced VEGF binding activity and/or therapeutic efficacy when stored at about 2 °C to about 8 °C for at least about twelve months. The amount of antibody monomers and/or antibody aggregates may be determined according to any technique suitable in the art, including those described or exemplified herein, including any one or combination of differential light scattering (DLS), differential scanning calorimetry (DSC), size exclusion chromatography (SE-HPLC), non-reducing and reducing capillary electrophoresis SDS (NR CE-SDS and R CE-SDS), and particulate count (PC).

In some aspects, the antibody formulation contains at least about 90% by weight of antibody monomers, and less than about 10% by weight of antibody aggregates with reduced VEGF binding activity and/or therapeutic efficacy when stored at about 2 °C to about 8 °C for at least about eighteen months. In some aspects, the antibody formulation contains at least about 93% by weight of antibody monomers, and less than about 7% by weight of antibody aggregates with reduced VEGF binding activity and/or therapeutic efficacy when stored at about 2 °C to about 8 °C for at least about eighteen months. In some aspects, the antibody formulation contains at least about 95% by weight of antibody monomers, and less than about 5% by weight of antibody aggregates with reduced VEGF binding activity and/or therapeutic efficacy when stored at about 2 °C to about 8 °C for at least about eighteen months. In some aspects, the antibody formulation contains at least about 96% by weight of antibody aggregates with reduced VEGF binding activity and/or therapeutic efficacy when stored at about 2 °C to about 8 °C for at least about eighteen months. In some aspects, the antibody formulation contains at least about 97% by weight of antibody monomers, and less than about 3% by weight of antibody aggregates with reduced VEGF binding activity and/or therapeutic efficacy when stored at about 2 °C to about 8 °C for at least about eighteen months. In some aspects, the antibody formulation contains at least about 98% by weight of antibody monomers, and less than about 2% by weight of antibody aggregates with reduced VEGF binding activity and/or therapeutic efficacy when stored at about 2 °C to about 8 °C for at least about eighteen months. In some aspects, the antibody formulation contains at least about 99% by weight of antibody monomers, and less than about 1% by weight of antibody aggregates with reduced VEGF binding activity and/or therapeutic efficacy when stored at about 2 °C to about 8 °C for at least about eighteen months. The amount of antibody monomers and/or antibody aggregates may be determined according to any technique suitable in the art, including those described or exemplified herein, including any one or combination of differential light scattering (DLS), differential scanning calorimetry (DSC), size exclusion chromatography (SE-HPLC), non-reducing and reducing capillary electrophoresis SDS (NR CE-SDS and R CE-SDS), and particulate count (PC).

In some aspects, the antibody formulation contains at least about 90% by weight of antibody monomers, and less than about 10% by weight of antibody aggregates with reduced VEGF binding activity and/or therapeutic efficacy when stored at about 2 °C to about 8 °C for at least about twenty-four months. In some aspects, the antibody formulation contains at least about 93% by weight of antibody monomers, and less than about 7% by weight of antibody aggregates with reduced VEGF binding activity and/or therapeutic efficacy when stored at about 2 °C to about 8 °C for at least about twenty-four months. In some aspects, the antibody formulation contains at least about 95% by weight of antibody monomers, and less than about 5% by weight of antibody aggregates with reduced VEGF binding activity and/or therapeutic efficacy when stored at about 2 °C to about 8 °C for at least about twenty-four months. In some aspects, the antibody formulation contains at least about 96% by weight of antibody aggregates with reduced VEGF binding activity and/or therapeutic efficacy when stored at about 2 °C to about 8 °C for at least about twenty-four months. In some aspects, the antibody formulation contains at least about 97% by weight of antibody monomers, and less than about 3% by weight of antibody aggregates with reduced VEGF binding activity and/or therapeutic efficacy when stored at about 2 °C to about 8 °C for at least about twenty-four months. In some aspects, the antibody formulation contains at least about 98% by weight of antibody monomers, and less than about 2% by weight of antibody aggregates with reduced VEGF binding activity and/or therapeutic efficacy when stored at about 2 °C to about 8 °C for at least about twenty-four months. In some aspects, the antibody formulation contains at least about 99% by weight of antibody monomers, and less than about 1% by weight of antibody aggregates with reduced VEGF binding activity and/or therapeutic efficacy when stored at about 2 °C to about 8 °C for at least about twenty-four months. The amount of antibody monomers and/or antibody aggregates may be determined according to any technique suitable in the art, including those described or exemplified herein, including any one or combination of differential light scattering (DLS), differential scanning calorimetry (DSC), size exclusion chromatography (SE-HPLC), non-reducing and reducing capillary electrophoresis SDS (NR CE-SDS and R CE-SDS), and particulate count (PC).

The present disclosure also features methods for treating a tumor in a subject in need thereof by administering a therapeutically effective amount of any of the antibody formulations described or exemplified herein. Preferably, the antibody formulations are used in methods for treating cancers such as platinum-resistant recurrent epithelial ovarian, fallopian tube, or primary peritoneal cancer, persistent, recurrent, or metastatic cervical cancer, metastatic colorectal cancer, metastatic HER2 negative breast cancer, metastatic renal cell carcinoma, glioblastoma, or non-small cell lung cancer (NSCLC). Therapeutic efficacy is attained, for example, by the bevacizumab antibody present in the administered formulation. Administration of the antibody formulation may be according to any suitable route, preferably by injection, and more preferably by intravenous injection. Administration may be carried out under the direction or supervision of a medical practitioner.

The antibody formulations described and exemplified herein may be for use as a medicament. The antibody formulations described and exemplified herein may be for use in the manufacture of a medicament for the treatment of one or more of a cancer such as platinum-resistant recurrent epithelial ovarian, fallopian tube, or primary peritoneal cancer, persistent, recurrent, or metastatic cervical cancer, metastatic colorectal cancer, metastatic HER2 negative breast cancer, metastatic renal cell carcinoma, glioblastoma, or non-small cell lung cancer (NSCLC). The formulations may be for use in the treatment of platinum-resistant recurrent epithelial ovarian, fallopian tube, or primary peritoneal cancer. The formulations may be for use in the treatment of persistent, recurrent, or metastatic cervical cancer. The formulations may be for use in the treatment of metastatic colorectal cancer. The formulations may be for use in the treatment of metastatic HER2 negative breast cancer. The formulations may be for use in the treatment of metastatic renal cell carcinoma. The formulations may be for use in the treatment of glioblastoma. The formulations may be for use in the treatment of non-small cell lung cancer (NSCLC).

The present disclosure also provides features kits. The kits may be used, for example, to practice any of the methods described or exemplified herein. In some aspects, a kit comprises any antibody formulation described or exemplified herein, and instructions for using the antibody formulation in any of the methods or uses described or exemplified herein. The kit may comprise a device for injecting the antibody formulation into a subject, including but not limited to a syringe and needle, or catheter.

The instructions included with the kit may include instructions for administering the antibody formulation in a method for treating platinum-resistant recurrent epithelial ovarian, fallopian tube, or primary peritoneal cancer, including instructions for injecting the antibody formulation into a platinum-resistant recurrent epithelial ovarian, fallopian tube, or primary peritoneal cancer patient in need thereof. In some aspects, the instructions included with the kit may include instructions for administering the antibody formulation in a method for treating persistent, recurrent, or metastatic cervical cancer, including instructions for injecting the antibody formulation into a persistent, recurrent, or metastatic cervical cancer patient in need thereof. In some aspects, the instructions included with the kit may include instructions for administering the antibody formulation in a method for treating metastatic colorectal cancer, including instructions for injecting the antibody formulation into a metastatic colorectal cancer patient in need thereof. In some aspects, the instructions included with the kit may include instructions for administering the antibody formulation in a method for treating metastatic HER2 negative breast cancer, including instructions for injecting the antibody formulation into a metastatic HER2 negative breast cancer patient in need thereof. In some aspects, the instructions included with the kit may include instructions for administering the antibody formulation in a method for treating metastatic renal cell carcinoma, including instructions for injecting the antibody formulation into a metastatic renal cell carcinoma patient in need thereof. In some aspects, the instructions included with the kit may include instructions for administering the antibody formulation in a method for treating glioblastoma, including instructions for injecting the antibody formulation into a glioblastoma patient in need thereof. In some aspects, the instructions included with the kit may include instructions for administering the antibody formulation in a method for treating non-small cell lung cancer (NSCLC), including instructions for injecting the antibody formulation into a non-small cell lung cancer (NSCLC) patient in need thereof.

The following examples are provided to describe the invention in greater detail. They are intended to illustrate, not to limit, the invention.

### Example 1 - Materials and Methods

Introduction. Antibody ONS-1045 represents a biosimilar of bevacizumab, and has been reformulated for enhanced storage stability. It is believed that the buffered formulation may, at the very least, reduce aggregation of the antibody during long-term storage. It is believed that the buffered formulation may reduce both the non-covalent and covalent dimerization of the bevacizumab molecule. Bevacizumab marketed as Avastin® (Genentech, Inc.) is formulated in a sodium phosphate buffer, including trehalose as a stabilizer, and including a mild surfactant and an acidic pH of 6.2. The experimental approach described below included development work to reformulate bevacizumab for enhanced colloidal stability. Significant enhancement in stability and, particularly with respect to a reduction in aggregation, was attained by changing the buffer and the pH.

Dynamic Light Scattering (DLS). The DLS testing method used a Wyatt DynaPro™ Plate Reader to provide information on protein size distribution and overall colloidal stability in solution. Hydrodynamic radius provided information on the presence of aggregation and confirmation of the molecule's structure in solution. DLS testing provided an orthogonal measure of size distribution in solution under non-denaturing conditions.

Differential Scanning Calorimetry (DSC). Differential scanning calorimetry measured the melting transitions for the protein and, thus, provided information on protein thermal stability in solution. Calorimetry was performed using a GE VP Capillary DSC system. The protein was heated from 25 °C to 95 °C at an optimized scan rate allowing the melting transitions (Tm) to occur while the protein is unfolding. A buffer control was heated alongside the sample and used to calculate melting temperatures and transitions. The DSC profile was typical of antibodies and demonstrated that the protein folded into distinct domains.

Size Exclusion Chromatography (SE-HPLC). SE-HPLC was used to monitor antibody size variant distribution. The SE-HPLC testing method separates proteins based on size. Species eluting before the monomer peak were aggregates (HMWS) and peaks eluting after the monomer peak were degradants (LMWS).

Species were separated using a TSK3000SWxl 7.8mm x 300mm column (Tosoh Bioscience Cat# 08541), with a flow rate of 0.5mL/min and a run time of 30 minutes; column at ambient temperature. The mobile phase comprised 0.2M potassium phosphate and 0.25M potassium chloride and a pH of 6.2. There were two forms of sample injection - neat injection 10uL @ 25 mg/mL and dilute injection 100uL @ 0.5 mg/mL (Neat injection measures the total aggregates including reversible aggregates, dilute injection measures the dimers primarily of covalent nature). Dilute samples were diluted with the mobile phase A (0.2M potassium phosphate, 0.25M potassium chloride, pH 6.2) to 0.5mg/mL.

Samples were incubated for 24 hours prior to analysis at 30 °C. The autosampler temperature was maintained at 30 °C for the entire duration of the run. Data were analyzed at 280nm.

Cation Exchange Chromatography (CEX). Bevacizumab samples were diluted in mobile phase A and digested with carboxypeptidase B. Species were separated using a cation-exchange HPLC column. A gradient was performed with mobile phase A and mobile phase B using a flow rate of 0.5mL/minute. Column temperature was maintained at 40 °C and samples were maintained at 2-8 °C. Data was analyzed at 280nm.

Particulate Content (Fluid Imaging). The Fluid Imaging (FI) system is an integrated system for rapidly analyzing particles in a moving fluid. The system automatically counts, images, and analyzes the particles or cells in a sample or a continuous flow. In the FI system, the sample is drawn into the flow chamber by a pump. Using the laser in Autolmage Mode, the FI system monitored the light scatter of the passing particles. The camera was set to capture images synchronously at a user defined interval. The scatter detection values were then saved by VisualSpreadsheet (in addition to all other particle properties and the image). The computer and digital signal processor work together to initiate, retrieve and process images of the field of view.

Osmolality. An Osmometer was used to measure the osmolality of buffer and protein solutions by means of freezing-point measurement. It utilized high-precision thermisters to sense the sample temperature, to control the degree of super cooling and freeze induction, and to measure the freezing point of the sample. Sample requirement was 20µL per measurement.

Intrinsic Fluorescence. Intrinsic Fluorescence Spectroscopy is a non-invasive biophysical characterization method that provides information on the tertiary structure of the protein. This method measured the degree of unfolding of the protein structure. Intensity and maximum wavelength of a protein sample (for example tryptophan emission) were determined on the fluorescence spectrometer. Test 600µL of 0.1mg/mL protein solution per replicate. Emission scan: Excitation at 295nm, start at 310nm end at 450nm.

HUVEC Cell Based VEGF Neutralization Assay. The primary mechanism of action of the anti-angiogenesis monoclonal antibody bevacizumab is to bind to VEGF and prevent binding to its cognate receptor. In this way, bevacizumab neutralizes the ability of VEGF to induce endothelial cell proliferation; therefore, potency of an anti-VEGF antibody can be quantified by its ability to inhibit VEGF-induced proliferation of cells. In the HUVEC cell-based potency assay, fixed concentrations of VEGF are incubated with serially diluted drug. Bevacizumab binds to VEGF in a dose dependent manner, making VEGF unavailable for other binding interactions. This drug-VEGF cocktail is then added to HUVEC cells seeded in multi-well plates and further incubated for continued proliferation. During incubation, HUVEC cells proliferate in a VEGF concentration-dependent manner. At low drug concentrations, more VEGF is available and therefore proliferation is high and vice versa. Antibody dose-dependent inhibition of HUVEC cell proliferation is assessed by quantifying the number of viable cells at the end of incubation. The VEGF neutralization assay is a relative assay in which the potency of samples is measured relative to a reference standard. The assay consists of three independent assay plates. In each plate, the cell viability data of standard and samples are fit to 4P logistic models to generate sigmoidal curves with independent curve parameters using statistical software; standard and sample curve parameters are compared to assess curve parallelism and when deemed parallel, the relative potency of test articles is calculated. The final reported value is an average of three independent values that are within acceptable variability.

VEGF Binding Immunoassay. The primary mechanism of action of the anti-angiogenesis monoclonal antibody bevacizumab is to bind to VEGF and prevent binding to its cognate receptor, thereby inhibiting VEGF mediated mitogenic effects on vascular endothelial cells. This neutralization of VEGF by bevacizumab inhibits the angiogenesis process, which in turn suppresses tumor survival and progression. Therefore, potency of an anti-VEGF antibody can be quantified by measuring its binding to VEGF in an ELISA. In this assay, a fixed concentration of VEGF is first coated on multi-well plates. After blocking non-specific binding sites, the immobilized VEGF is reacted with serially diluted Reference standard and Test samples. The unbound antibody is washed away and the wells are incubated with horseradish peroxidase (HRP) conjugated anti-kappa light chain antibody which binds to the VEGF-Antibody complexes. Next, the unbound secondary antibody is washed away and the wells are incubated with 3,3',5,5'-Tetramethylbenzidine (TMB) HRP substrate to produce a colored product. The color development is quenched by adding phosphoric acid and the absorbance values are read. The optical density (O.D.) values obtained are directly proportional to the amount of sample bound to VEGF. The VEGF binding assay is a relative assay in which the potency of samples is measured relative to a reference standard. The assay consists of two independent assay plates. In each plate, the O.D. data of standard and samples are fit to 4P logistic models to generate sigmoidal curves with independent curve parameters using statistical software; standard and sample curve parameters are compared to assess curve parallelism and when deemed parallel, the relative potency of test articles is calculated. The final reported value is an average of two independent values that are within acceptable variability.

### Example 2 - Effect of Buffer, Stabilizers, and pH on Bevacizumab Conformational and Colloidal Stability

Initial experiments evaluated the buffer components for the conformational and colloidal stability of bevacizumab. It was determined that citrate, phosphate, and acetate buffers are ideal for stability of bevacizumab. Moreover, individually these buffers exhibited a protective effect towards aggregation of bevacizumab that is induced by heating or shaking related stress. Further experiments evaluated if a combination of these buffers (citrate, phosphate and acetate) exhibited superior stabilizing effects. A citrate phosphate buffer produced significantly lower aggregates (including covalent type dimers) and lower charge species relative to the sodium phosphate buffer in the bevacizumab match composition (matched to the formulation of the commercially available Avastin® formulation).

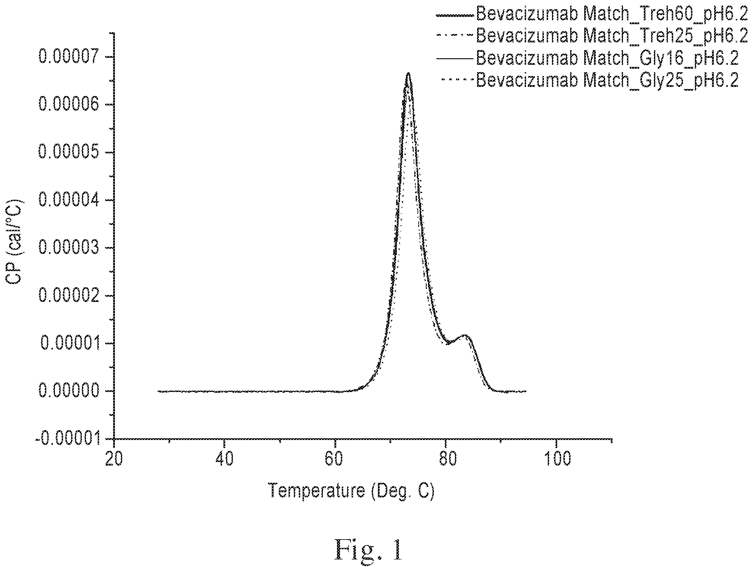

The effect of the trehalose stabilizer in a 50 mM sodium phosphate buffer was compared with alternative stabilizers, including sucrose, sorbitol, mannitol, and glycine. Conformational stability of the antibody in the different stabilized buffer composition was then assessed by DSC (Fig. 1). These data are presented in Table 1, and show that all of the stabilizers tested were equal to or better than trehalose.

**Table 1. Alternative conformational stabilizing agents in sodium phosphate buffer.**

| **Buffer Conditions** | **All conditions: 50mM sodium phosphate** | **Final Buffer pH** | **Tₘ₁** | **Tₘ₂** |
|---|---|---|---|---|
| 1 | Trehalose 60mg/mL (match) | 6.20 | 73.3 | 83.5 |
| 2 | Trehalose 25mg/mL | 6.26 | 73.0 | 83.0 |
| 3 | Sucrose 25mg/mL | 6.13 | 72.9 | 82.9 |
| 4 | Sucrose 60mg/mL | 6.11 | 73.4 | 83.4 |
| 5 | Sorbitol 25mg/mL | 6.19 | 73.1 | 82.9 |
| 6 | Sorbitol 60mg/mL | 6.12 | 73.6 | 83.5 |
| 7 | Mannitol 25mg/mL | 6.19 | 73.0 | 83.2 |
| 8 | Mannitol 60mg/mL | 6.05 | 73.7 | 83.4 |
| 9 | Glycine 16mg/mL | 6.11 | 73.5 | 83.3 |
| 10 | Glycine 25mg/mL | 6.05 | 73.9 | 83.6 |

The alternative stabilizers (sucrose, sorbitol, mannitol, and glycine) were next used with the citrate phosphate buffer, and the conformational stability of the bevacizumab antibody was assessed by DSC. The data are presented in Table 2, and show that the alternative stabilizers in a citrate phosphate buffer were equal to or better than the bevacizumab match formulation.

**Table 2. Alternative conformational stabilizing agents in citrate phosphate (C/P) buffer.**

| Buffer Condition | Sample | pH | Tm1 | Tm2 |
|---|---|---|---|---|
| 1 | Bevacizumab match: 50 mM sodium phosphate, | 6.20 | 73.3 | 83.5 |
| | Trehalose 60 mg/mL | | | |
| 2 | 50 mM C/P sucrose 60 gm/mL | 6.15 | 73.0 | 83.0 |
| 3 | 50 mM C/P sorbitol 60 mg/mL | 6.14 | 73.3 | 83.3 |
| 4 | 50 mM C/P mannitol 60 mg/mL | 6.11 | 73.3 | 83.2 |
| 5 | 50 mM C/P glycine 25 mg/mL | 6.1 | 73.5 | 83.6 |

Using the citrate phosphate buffer with trehalose as the stabilizing agent, the effect of pH on the conformational stability of the antibody (bevacizumab) was assessed. DSC was used to measure the antibody stability. The data, presented in Table 3, show that the unfolding temperatures for Bevacizumab as measured in each formulation compositions are comparable to that observed for the Bevacizumab match composition, but only at pH of greater than 5.6 (5.6, 5.8, 6.0, 6.2). At lower pH (particularly 5.0), an early unfolding event takes place at lower temperature of about 65 °C, thus making such lower pH (below 5.6) unsuitable for formulation of bevacizumab.

**Table 3. Effect of pH on bevacizumab thermal/conformational stability in a 50 mM citrate or 50 mM citrate phosphate buffer.**

| **Sample** | **Final Buffer pH** | **Tm1** | **Tm2** | **Tm3** |
|---|---|---|---|---|
| Bevacizumab Match | 6.20 | 73.4 | 83.5 | |
| 35mM Cit Treh 60mg/mL pH 5.8 | 5.79 | 72.3 | 83.0 | |
| 50mM Cit Treh 60mg/mL pH5.0 | 4.99 | 65.3 | 71.1 | 79.6 |
| 50mM Cit Treh 60mg/mL pH5.2 | 5.13 | 71.5 | 80.9 | |
| 50mM Cit Treh 60mg/mL pH5.4 | 5.32 | 71.9 | 82.0 | |
| 50mM Cit Treh 60mg/mL pH5.6 | 5.51 | 72.1 | 82.4 | |
| 50mM Cit Treh 60mg/mL pH 5.8 | 5.70 | 72.2 | 82.8 | |
| 50mM Cit Treh 60mg/mL pH6.0 | 5.93 | 72.3 | 82.8 | |
| 50mM Cit Treh 60mg/mL pH6.2 | 6.13 | 72.4 | 83.0 | |
| 50mM C/P Treh 60mg/mL pH5.0 | 5.08 | 72.2 | 81.2 | |
| 50mM C/P Treh 60mg/mL pH5.2 | 5.25 | 72.5 | 81.9 | |
| 50mM C/P Treh 60mg/mL pH5.4 | 5.43 | 72.8 | 82.3 | |
| 50mM C/P Treh 60mg/mL pH5.6 | 5.66 | 72.8 | 83.1 | |
| 50mM C/P Treh 60mg/mL pH5.8 | 5.92 | 72.9 | 83.1 | |
| 50mM C/P Treh 60mg/mL pH6.0 | 6.13 | 73.0 | 83.2 | |
| 50mM C/P Treh 60mg/mL pH6.2 | 6.26 | 73.1 | 83.4 | |

Parallel experiments evaluated acetate as the buffering agent. Acetate was assessed at concentrations of 5 mM, 15 mM, and 25 mM, with variable pH (Tables 4 and 5). These experiments compared sucrose (60 mg/ml) and trehalose (60 mg/ml) as the stabilizing agent. Stability of the bevacizumab molecule in each composition was then assessed by DSC. The data are shown in Tables 4 and 5. It was observed that increasing the molarity of acetate lowered the Tm, and increasing the pH also lowered the Tm (Tables 4 and 5). Enhanced conformational stability was shown for pH 5.6 and 5.8 (Table 5).

**Table 4. Conformational stability of bevacizumab in acetate trehalose buffered formulations.**

| **Sample** | **Final pH** | **Tm1** | **Tm2** |
|---|---|---|---|
| Bevacizumab Match 50mM Phosphate pH6.2 | 6.20 | 73.4 | 83.5 |
| 5mM Acetate 159mM Treh. pH5.6 | 5.51 | 74.2 | 83.3 |
| 15mM Acetate 159mM Treh. pH5.6 | 5.52 | 73.9 | 83.3 |
| 25mM Acetate 159mM Treh. pH5.6 | 5.56 | 73.8 | 83.0 |
| 5mM Acetate 159mM Treh. pH5.8 | 5.78 | 74.1 | 83.5 |
| 15mM Acetate 159mM Treh. pH5.8 | 5.77 | 74.0 | 83.3 |
| 25mM Acetate 159mM Treh. pH5.8 | 5.76 | 73.8 | 83.3 |
| 5mM Acetate 159mM Treh. pH6.0 | 5.93 | 74.1 | 83.6 |
| 15mM Acetate 159mM Treh. pH6.0 | 5.91 | 73.9 | 83.4 |
| 25mM Acetate 159mM Treh. pH6.0 | 5.97 | 73.8 | 83.7 |
| 5mM Acetate 159mM Treh. pH6.2 | 6.11 | 74.1 | 83.8 |
| 15mM Acetate 159mM Treh. pH6.2 | 6.15 | 73.8 | 83.8 |
| 25mM Acetate 159mM Treh. pH6.2 | 6.14 | 73.6 | 83.8 |

**Table 5. Conformational stability of bevacizumab in acetate sucrose buffered formulations.**

| **Sample** | **Actual Buffer pH** | **Final pH** @ **0.5mg/mL** | **Tm1** | **Tm2** |
|---|---|---|---|---|
| Bevacizumab Match 50mM Phosphate pH6.2 | 6.20 | 6.20 | 73.4 | 83.7 |
| 5mM Acetate 60mg/mL Suc pH5.6 | 5.57 | 5.72 | 74.1 | 83.4 |
| 15mMAcetate 60mg/mL Suc pH5.6 | 5.52 | 5.60 | 73.9 | 83.0 |
| 5mM Acetate 60mg/mL Suc pH5.8 | 5.76 | 5.92 | 74.1 | 83.4 |
| 15mM Acetate | 5.73 | 5.80 | 73.9 | 83.3 |
| 60mg/mL Suc pH5.8 | | | | |

### Example 3 - Storage Stability in Citrate Phosphate Buffered Trehalose and Acetate Sucrose Formulations

Four buffered formulations were selected to assess long-term storage stability of the bevacizumab molecule over 18 months. These formulations were run in parallel with the bevacizumab match/reference formulation (condition 1). Storage conditions were as follows: antibody at -25 mg/ml (neat) or diluted; storage at 5 °C, 30 °C, or 37 °C; shaking at 150 RPM at room temperature; and freeze/thaw (20 °C to room temperature, for three cycles). The formulations tested are listed below:
Condition 1: Bevacizumab (Avastin®) Match
   50 mM Sodium Phosphate
   159 mM Trehalose
   0.04% polysorbate 20
   pH 6.20
   Q.S. with Sterile water for injection
Condition 2: Bevacizumab Citrate Phosphate, pH 5.8
   50 mM Citrate Phosphate
   159 mM Trehalose
   0.04% Polysorbate 20
   pH 5.80
   Q.S. with Sterile Water for injection
Condition 3: Bevacizumab Citrate Phosphate, pH 6.0
   50 mM Citrate Phosphate
   159 mM Trehalose
   0.04% Polysorbate 20
   pH 6.0
   Q.S. with Sterile Water for injection
Condition 4: Bevacizumab Acetate, pH 5.6
   15 mM Acetate
   175 mM Sucrose
   0.04% Polysorbate 20
   pH 5.60
   Q.S. with Sterile Water for injection
Condition 5: Bevacizumab Acetate, pH 5.8
   15 mM Acetate
   175 mM Sucrose
   0.04% Polysorbate 20
   pH 5.80
   Q.S. with Sterile Water for injection

Stability of the antibody under each storage condition was tested by a battery of routine analytical and extended characterization assays, including but not limited, to size exclusion chromatography (SEC), cation exchange chromatography (CEX), CE-SDS, HUVEC cell based VEGF neutralization assay, VEGF binding immunoassay and particulate count (PC). Size exclusion chromatography was used to assess the percentage of antibody monomers, the percentage of total aggregates (covalent and non-covalent), and the percentage of degradants. The comparative stability of both formulation types were assessed along with the bevacizumab (Avastin®) reference/match composition. Samples on long term storage stability stored at 5 °C ±3 °C over 18 months, indicate that both the citrate phosphate based compositions (Conditions 2 and 3) and the acetate buffer based compositions (Conditions 4 and 5) are more stable than Avastin® match composition (Fig. 2A; Fig. 2A (i); Fig 2A (ii); Table 6). Figure 2B, Figure 2B (i), Figure 2B (ii) and Table 7 indicate the measured covalent dimers is present in all five bevacizumab biosimilar compositions, however Conditions 2, 3, 4 and 5 have lower covalent dimers than those present in the bevacizumab match composition (condition 1) (Table 7).

**Table 6. Total aggregates in bevacizumab biosimilar formulations (long term stability at 5 °C) as measured by SE-HPLC (neat injection).**

| Formulation Composition | % Aggregates | | | | |
|---|---|---|---|---|---|
| Time (at 5°C) | Condition 1 | Condition 2 | Condition 3 | Condition 4 | Condition 5 |
| T0 | 6.0 | 3.1 | 3.5 | 5.7 | 6.2 |
| 2 Months | 6.3 | 3.4 | 3.8 | 3.4 | 4.9 |
| 3.5 Months | 6.3 | 3.3 | 3.9 | 3.4 | 4.9 |
| 7 Months | 6.5 | 3.5 | 4.0 | 3.6 | 4.9 |
| 12 Months | 6.4 | 3.4 | 4.0 | 3.0 | 4.1 |
| 18 Months | 7.0 | 3.9 | 4.3 | 3.4 | 4.6 |

**Table 7. Covalent dimers in bevacizumab biosimilar formulations (long term stability) as measured by SE-HPLC (dilute injection).**

| Formulation Composition | % Covalent Dimer | | | | |
|---|---|---|---|---|---|
| Time (at 5°C) | Condition 1 | Condition 2 | Condition 3 | Condition 4 | Condition 5 |
| T0 | 1.7 | 1.7 | 1.7 | 1.5 | 1.4 |
| 3.5 Months | 2.0 | 2.4 | 1.9 | 2.3 | 2.0 |
| 7 Months | 2.5 | 2.2 | 2.5 | 2.1 | 2.2 |
| 12 Months | 2.4 | 2.1 | 2.1 | 1.7 | 1.9 |
| 18 Months | 2.9 | 2.5 | 2.6 | 2.1 | 2.3 |

Acidic charged species as measured by cation exchange chromatography (CEX) were also tested for all samples during the 18-month long storage stability study (Table 7 (i); Figure 2B (iii); Figure 2B (iv) and Figure 2B (v)). Across all 5 compositions, the charged species particularly the acidic charged species did not vary significantly over the 18 months of storage at 5 °C.

**Table 7 (i). % Acidic Species in bevacizumab biosimilar formulations (long term stability) as measured by CEX-HPLC.**

| Formulation Composition | % Acidic Species | | | | |
|---|---|---|---|---|---|
| Time (at 5°C) | Condition 1 | Condition 2 | Condition 3 | Condition 4 | Condition 5 |
| T0 | 22.9 | 25.7 | 26.5 | 26.9 | 26.7 |
| 3.5 month | 28.3 | 27.9 | 28.1 | 28.4 | 27.9 |
| 7 month | 29.2 | 28.8 | 29.1 | 29.4 | 29.4 |
| 12 month | 29.7 | 28.6 | 29.0 | 29.5 | 28.9 |
| 18 month | 28.7 | 27.3 | 27.3 | 27.6 | 27.4 |

The relative potency for Adalimumab biosimilar formulations as measured by HUVEC cell based VEGF neutralization assay were found to be within 90 - 110 % for all conditions (condition 2-5) as compared to Condition 1 (Table7 (ii)). This finding also confirms that the potency of the formulations (condition 2-5) is not affected due to alterations in formulation composition and is equivalent to the formulation of Avastin composition over 18 months of storage at 2-8 °C.

**Table 7 (ii). Relative potency for bevacizumab biosimilar formulations (conditions 1 to 5) as measured by the HUVEC cell based VEGF neutralization assay after 18 months of storage at 2-8 °C.**

| **Sample Description** | **Relative Potency Compared to Condition 1** |
|---|---|
| Condition 1 | 100 |
| Condition 2 | 103 |
| Condition 3 | 101 |
| Condition 4 | 100 |
| Condition 5 | 99 |

The relative potency for Adalimumab biosimilar formulations as measured by VEGF binding Immunoassay were found to be within 90 - 100 % for all conditions (condition 2-5) as compared to Condition 1 (Table 7(iii)). This finding also confirms that the potency of the formulations (condition 2-5) is not affected due to alterations in formulation composition and is equivalent to the formulation of Avastin composition over 18 months of storage at 2-8 °C.

**Table 7 (iii). Relative potency for bevacizumab biosimilar formulations (conditions 1 to 5) as measured by the VEGF binding Immunoassay after 18 months of storage at 2-8 °C.**

| **Sample Description** | **Relative Potency Compared to Condition 1** |
|---|---|
| Condition 1 | 100% |
| Condition 2 | 95% |
| Condition 3 | 93% |
| Condition 4 | 97% |
| Condition 5 | 97% |

Samples on accelerated storage stability (30°C) indicate that both the citrate phosphate based compositions and the acetate buffer based compositions are more stable than the bevacizumab (Avastin®) match composition (Fig. 2C and Fig. 2D; Tables 8 and 9). Figure 2D and Table 9 indicate there are measurable covalent dimers in all five bevacizumab biosimilar compositions. All biosimilar formulation conditions (2-5) were found to have lower covalent dimers than those present in the bevacizumab match composition.

**Table 8. Total aggregates in bevacizumab biosimilar formulations (accelerated stability at 30 °C) as measured by SE-HPLC (neat injection).**

| Formulation Composition | % Aggregates | | | | |
|---|---|---|---|---|---|
| Time (at 30°C) | Condition 1 | Condition 2 | Condition 3 | Condition 4 | Condition 5 |
| T0 | 6.0 | 3.1 | 3.5 | 5.7 | 6.2 |
| Day 7 | 6.1 | 3.3 | 3.3 | 3.8 | 5.3 |
| Day 14 | 6.6 | 3.5 | 4.1 | 3.9 | 5.7 |
| 3.5 Months | 8.3 | 4.6 | 5.3 | 4.1 | 6.2 |

**Table 9. Covalent dimers in bevacizumab biosimilar formulations (accelerated stability@30 °C) as measured by SE-HPLC (dilute injection).**

| Formulation Condition | % Covalent Dimer | | | | |
|---|---|---|---|---|---|
| Time (at° 30C) | Condition 1 | Condition 2 | Condition 3 | Condition 4 | Condition 5 |
| T0 | 1.7 | 1.7 | 1.7 | 1.5 | 1.4 |
| 2 Months | 3.6 | 2.8 | 3.0 | 2.3 | 2.5 |
| 3.5 Months | 4.0 | 3.2 | 3.7 | 2.7 | 3.2 |

Samples on accelerated storage stability (37 °C) indicated that both the citrate phosphate based compositions and the acetate buffer based compositions are more stable than the bevacizumab match composition (Fig. 2E and Fig. 2F; Tables 10 and 11). Figure 2F and Table 11 indicate the presence of measurable covalent dimers in all five bevacizumab biosimilar compositions. Nevertheless, all conditions (2-5), have lower covalent dimers than those present in the bevacizumab match composition.

**Table 10. Total aggregates in bevacizumab biosimilar formulations (Accelerated stability at 37 °C) as measured by SE-HPLC (neat injection).**

| Formulation Composition | % Aggregates | | | | |
|---|---|---|---|---|---|
| Time (at 37°C) | Condition 1 | Condition 2 | Condition 3 | Condition 4 | Condition 5 |
| T0 | 6.0 | 3.1 | 3.5 | 5.7 | 6.2 |
| Day 7 | 6.7 | 3.4 | 4.0 | 3.9 | 5.7 |
| Day 14 | 7.3 | 3.9 | 4.6 | 4.0 | 6.2 |
| Day 21 | 7.8 | 4.2 | 4.9 | 4.3 | 6.2 |
| Day 28 | 8.3 | 4.4 | 5.1 | 4.2 | 5.6 |
| 2 Months | 9.9 | 5.2 | 5.9 | 4.6 | 6.4 |

**Table 11. Covalent dimers in bevacizumab biosimilar formulations (accelerated stability at 37 °C) as measured by SE-HPLC (dilute injection).**

| Formulation Composition | % Covalent Dimer | | | | |
|---|---|---|---|---|---|
| Time (at 37°C) | Condition 1 | Condition 2 | Condition 3 | Condition 4 | Condition 5 |
| T0 | 1.7 | 1.7 | 1.7 | 1.5 | 1.4 |
| 2 Months | 5.1 | 3.7 | 3.8 | 3.0 | 3.2 |

Samples on stress testing (shaking at room temperature at 150 rpm), indicate that both the citrate phosphate based compositions and the acetate buffer based compositions are more stable than the bevacizumab (Avastin®) match composition (Fig. 2G and Fig. 2H; Tables 12 and 13). The Acetate-Sucrose composition (condition 5) had a slightly higher percentage of aggregate, indicating that the pH of 5.6 is preferred for formulation stability of bevacizumab over pH 5.8. Figure 2H and Table 13 indicate the measured covalent dimers in all five bevacizumab biosimilar compositions. All conditions (2-5), were observed to have lower covalent dimers than those present in the bevacizumab (Avastin®) match composition.

**Table 12. Total aggregates in bevacizumab biosimilar formulations (shaking at 150 rpm) as measured by SE-HPLC (neat injection).**

| Formulation Composition Time (shaking at 150 rpm) | % Aggregates | | | | |
|---|---|---|---|---|---|
| | Condition 1 | Condition 2 | Condition 3 | Condition 4 | Condition 5 |
| T0 | 6.0 | 3.1 | 3.5 | 5.7 | 6.2 |
| Day 7 | 6.4 | 3.3 | 3.9 | 4.0 | 5.3 |
| Day 14 | 6.5 | 3.2 | 4.0 | 4.0 | 6.0 |
| Day 21 | 6.7 | 3.8 | 4.3 | 4.2 | 5.7 |

**Table 13. Covalent dimers in bevacizumab biosimilar formulations (shaking at 150 rpm) as measured by SE-HPLC (dilute injection).**

| Formulation Composition Time (shaking at 150 rpm) | % Covalent Dimer | | | | |
|---|---|---|---|---|---|
| | Condition 1 | Condition 2 | Condition 3 | Condition 4 | Condition 5 |
| T0 | 1.7 | 1.7 | 1.7 | 1.5 | 1.4 |
| Day 21 | 2.8 | 2.2 | 2.4 | 2.0 | 2.1 |

Samples on stress testing (freeze/thaw testing) indicate that both the citrate phosphate based compositions and the acetate buffer based compositions are equivalent to the bevacizumab (Avastin®) match composition with regard to offering protection against freeze/thaw stress (Fig. 2I and Fig. 2J; Tables 14 and 15). The Acetate-Sucrose composition (condition 5) had a slightly higher percentage of aggregate, indicating that the pH of 5.6 is preferred for formulation stability of bevacizumab over pH 5.8. Figure 2J and Table 15 indicate the presence of measurable covalent dimers in all five bevacizumab biosimilar compositions. All conditions (2-5), had lower covalent dimers than those present in the bevacizumab match composition.

**Table 14. Total aggregates in bevacizumab biosimilar formulations (shaking at 150 rpm) as measured by SE-HPLC (neat injection).**

| Formulation Composition Freeze/thaw stress testing | % Aggregates | | | | |
|---|---|---|---|---|---|
| | Condition 1 | Condition 2 | Condition 3 | Condition 4 | Condition 5 |
| T0 | 6.0 | 3.1 | 3.5 | 5.7 | 6.2 |
| cycle 1 RT/-20°C | 6.1 | 3.1 | 3.8 | 3.7 | 5.2 |
| cycle 3 RT/-20°C | 6.2 | 3.2 | 3.7 | 3.6 | 5.8 |

**Table 15. Covalent dimers in bevacizumab biosimilar formulations (Freeze/thaw stress) as measured by SE-HPLC (dilute injection).**

| Formulation conditions Freeze/thaw stress testing | % Covalent Dimer | | | | |
|---|---|---|---|---|---|
| | Condition 1 | Condition 2 | Condition 3 | Condition 4 | Condition 5 |
| T0 | 1.7 | 1.7 | 1.7 | 1.5 | 1.4 |
| cycle 1 RT/-20°C | 1.7 | 1.7 | 1.7 | 1.6 | 1.6 |
| cycle 3 RT/-20°C | 1.7 | 1.6 | 1.8 | 1.6 | 1.7 |

### Example 4 - Biophysical Properties of Bevacizumab Biosimilar in Citrate Phosphate Buffered Trehalose and Acetate Sucrose Formulations

Biophysical properties of the four buffered biosimilar test formulations were assessed in parallel with the bevacizumab match reference formulation. Biophysical properties including but not limited to those tested by Differential Scanning Calorimetry (DSC), Dynamic light scattering (DLS), Fluorescence Spectroscopy (Int. Fl.) were assessed. Similarity of biosimilars was assessed by several orthogonal tools, with these biophysical methods being one such approach within the orthogonal analytical methods to assess biosimilarity. Orthogonal tools indicate the biophysical properties of four buffered formulations of bevacizumab biosimilar (condition 2-5) were similar or better to that of bevacizumab match composition (condition 1).

The melting temperatures and, hence, the thermal unfolding pattern for bevacizumab in all formulations is similar with a Tm1 of about 73 °C and Tm2 of about 83 °C (Table 16). This indicates that all the developed formulation conditions offer similar conformational stability to bevacizumab. Ultimately it is the long term stability study (18 months storage at 5 °C) as described in Example 3, which conclusively identifies conditions 2-5 as formulations (*i*.*e*. compositions) wherein Bevacizumab is more stable and less prone to aggregation.

**Table 16. Conformational stability of bevacizumab biosimilar formulations compositions as measured by DSC.**

| **Formulation Condition** | **Sample** | **Actual Buffer pH** | **Tm1** | **Tm2** |
|---|---|---|---|---|
| 1 | Bevacizumab Match | 6.2 | 73.3 | 83.3 |
| 2 | Bevacizumab Citrate Phosphate pH 5.8 | 5.8 | 72.8 | 82.8 |
| 3 | Bevacizumab Citrate Phosphate pH 6.0 | 6.0 | 72.4 | 82.9 |
| 4 | Bevacizumab Acetate pH 5.6 | 5.6 | 73.5 | 83.1 |
| 5 | Bevacizumab Acetate 5.8 | 5.8 | 73.6 | 83.3 |

Dynamic Light Scattering (DLS) based assessment of the hydrodynamic properties of bevacizumab biosimilar in all four formulation conditions was assessed in comparison to bevacizumab match composition (condition 1). The hydrodynamic radius of bevacizumab biosimilar increases from about 6 nm to 7 nm (at a concentration of 15 mg/ml) in conditions 1-3 (Table 17, Figure 3). The acetate conditions, 4 and 5, showed notable different size trends as compared to the bevacizumab match composition (condition 1), indicating a better colloidal stability. While the hydrodynamic size in the citrate phosphate conditions, 2 and 3, the trend was similar to the bevacizumab match condition (condition 1), the reversible aggregate and covalent dimer formation trends (long term storage over a duration of 18 months and accelerated temperature storage stability as described in example 3) indicate it offers better protection against aggregation.

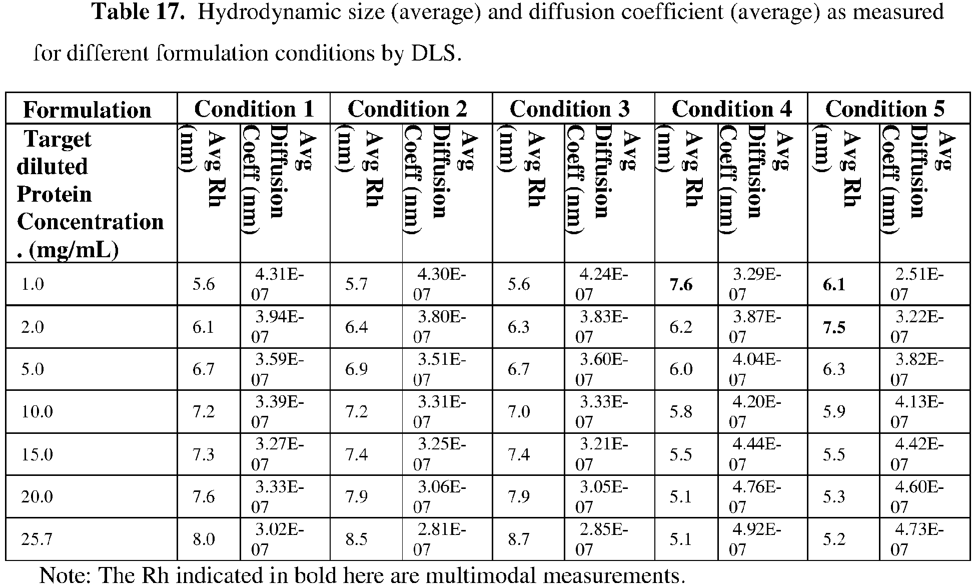

Intrinsic fluorescence spectroscopy indicates all formulation conditions offering similar conformational stability to bevacizumab as the match composition (condition 1) (Table 18 and Fig. 4). The key biophysical descriptors of this test (absorbance maximum and wavelength maximum) are similar for all formulation conditions.

**Table 18. Intrinsic fluorescence spectra for all formulation conditions indicating the average peak maximum and the absorbance**

| | | Tryptophan - 295/310 | |
|---|---|---|---|
| Formulation Condition | Formulation Description | Average Peak max (nm) | Average Absorbance |
| 1 | Bevacizumab Match | 341 | 542.6 |
| 2 | Bevacizumab Citrate Phosphate pH 5.8 | 338 | 502.7 |
| 3 | Bevacizumab Citrate Phosphate pH 6.0 | 336 | 522.3 |
| 4 | Bevacizumab Acetate pH 5.6 | 337 | 518.5 |
| 5 | Bevacizumab Acetate pH 5.8 | 336 | 557.6 |

## Claims

1. A buffered antibody formulation, comprising from 15 mg/ml to 35 mg/ml of an antibody comprising a heavy chain comprising the amino acid sequence of SEQ ID NO: 1 and a light chain comprising the amino acid sequence of SEQ ID NO: 2, a buffer comprising from 5 mM to 25 mM of sodium acetate, from 150 mM to 201 mM of sucrose, and from 0.03% (v/v) to 0.05% (v/v) of polysorbate 20, wherein the antibody formulation has a pH of from 5.6 to 5.8, wherein the antibody formulation is stable for at least 18 months when stored under refrigerated conditions at 5 °C.

2. The formulation of claim 1, wherein the formulation comprises:
from 20 mg/ml to 30 mg/ml of the antibody,
from 23 mg/ml to 27 mg/ml of the antibody,
from 24 mg/ml to 27 mg/ml of the antibody,
from 24 mg/ml to 26 mg/ml of the antibody, or
from 25 mg/ml to 26 mg/ml of the antibody.

3. The formulation of claim 1 or claim 2, wherein the formulation comprises 25.5 mg/ml of the antibody.

4. The formulation of claim 1 or claim 2, wherein the formulation comprises 25 mg/ml of the antibody.

5. The formulation of any one of the preceding claims, wherein the buffer comprises from 11 mM to 19 mM of sodium acetate.

6. The formulation of any one of the preceding claims, wherein the buffer comprises from 13 mM to 17 mM of sodium acetate.

7. The formulation of any one of the preceding claims, wherein the buffer comprises from 13 mM to 16 mM of sodium acetate.

8. The formulation of any one of the preceding claims, wherein the buffer comprises 15 mM of sodium acetate.

9. The formulation of any one of the preceding claims, wherein the formulation comprises from 165 mM to 185 mM of sucrose.

10. The formulation of any one of the preceding claims, wherein the formulation comprises from 170 mM to 180 mM of sucrose.

11. The formulation of any one of the preceding claims, wherein the formulation comprises from 174 mM to 176 mM of sucrose.

12. The formulation of any one of the preceding claims, wherein the formulation comprises 175 mM of sucrose.

13. The formulation of any one of the preceding claims, wherein the formulation comprises 0.04% (v/v) of polysorbate 20.

14. The formulation of any one of the preceding claims, wherein the formulation has a pH of 5.8, or a pH of 5.6.

## Patentansprüche

1. Gepufferte Antikörperformulierung, umfassend von 15 mg/ml bis 35 mg/ml eines Antikörpers, umfassend eine schwere Kette, die die Aminosäuresequenz von SEQ ID Nr. 1 umfasst, und eine leichte Kette, die die Aminosäuresequenz von SEQ ID Nr. 2 umfasst, einen Puffer, der von 5 mM bis 25 mM Natriumacetat umfasst, von 150 mM bis 201 mM Saccharose und von 0,03 Vol.-% bis 0,05 Vol.-% Polysorbat 20, wobei die Antikörperformulierung einen pH-Wert von 5,6 bis 5,8 aufweist, wobei die Antikörperformulierung für mindestens 18 Monate stabil ist, wenn sie unter gekühlten Bedingungen bei 5 °C gelagert wird.

2. Formulierung nach Anspruch 1, wobei die Formulierung umfasst:
von 20 mg/ml bis 30 mg/ml des Antikörpers,
von 23 mg/ml bis 27 mg/ml des Antikörpers,
von 24 mg/ml bis 27 mg/ml des Antikörpers,
von 24 mg/ml bis 26 mg/ml des Antikörpers oder
von 25 mg/ml bis 26 mg/ml des Antikörpers.

3. Formulierung nach Anspruch 1 oder 2, wobei die Formulierung 25,5 mg/ml des Antikörpers umfasst.

4. Formulierung nach Anspruch 1 oder 2, wobei die Formulierung 25 mg/ml des Antikörpers umfasst.

5. Formulierung nach einem der vorhergehenden Ansprüche, wobei der Puffer von 11 mM bis 19 mM Natriumacetat umfasst.

6. Formulierung nach einem der vorhergehenden Ansprüche, wobei der Puffer von 13 mM bis 17 mM Natriumacetat umfasst.

7. Formulierung nach einem der vorhergehenden Ansprüche, wobei der Puffer von 13 mM bis 16 mM Natriumacetat umfasst.

8. Formulierung nach einem der vorhergehenden Ansprüche, wobei der Puffer 15 mM Natriumacetat umfasst.

9. Formulierung nach einem der vorhergehenden Ansprüche, wobei die Formulierung von 165 mM bis 185 mM Saccharose umfasst.

10. Formulierung nach einem der vorhergehenden Ansprüche, wobei die Formulierung von 170 mM bis 180 mM Saccharose umfasst.

11. Formulierung nach einem der vorhergehenden Ansprüche, wobei die Formulierung von 174 mM bis 176 mM Saccharose umfasst.

12. Formulierung nach einem der vorhergehenden Ansprüche, wobei die Formulierung 175 mM Saccharose umfasst.

13. Formulierung nach einem der vorhergehenden Ansprüche, wobei die Formulierung 0,04 Vol.-% Polysorbat 20 umfasst.

14. Formulierung nach einem der vorhergehenden Ansprüche, wobei die Formulierung einen pH-Wert von 5,8 oder einen pH-Wert von 5,6 aufweist.

## Revendications

1. Formulation d'anticorps tamponnée comprenant de 15 mg/ml à 35 mg/ml d'un anticorps comprenant une chaîne lourde qui comprend la séquence d'acides aminés SEQ ID N° : 1 et une chaîne légère qui comprend la séquence d'acides aminés SEQ ID N° : 2, un tampon comprenant de 5 mM à 25 mM d'acétate de sodium, de 150 mM à 201 mM de sucrose et de 0,03 % (v/v) à 0,05 % (v/v) de Polysorbate 20, où la formulation d'anticorps a un pH qui va de 5,6 à 5,8, où la formulation d'anticorps est stable pendant au moins 18 mois quand elle est conservée dans des conditions réfrigérées à 5 °C.

2. Formulation de la revendication 1, où la formulation comprend :
de 20 mg/ml à 30 mg/ml de l'anticorps,
de 23 mg/ml à 27 mg/ml de l'anticorps,
de 24 mg/ml à 27 mg/ml de l'anticorps,
de 24 mg/ml à 26 mg/ml de l'anticorps ou
de 25 mg/ml à 26 mg/ml de l'anticorps.

3. Formulation de la revendication 1 ou de la revendication 2, où la formulation comprend 25,5 mg/ml de l'anticorps.

4. Formulation de la revendication 1 ou de la revendication 2, où la formulation comprend 25 mg/ml de l'anticorps.

5. Formulation de l'une quelconque des revendications précédentes, où le tampon comprend de 11 mM à 19 mM d'acétate de sodium.

6. Formulation de l'une quelconque des revendications précédentes, où le tampon comprend de 13 mM à 17 mM d'acétate de sodium.

7. Formulation de l'une quelconque des revendications précédentes, où le tampon comprend de 13 mM à 16 mM d'acétate de sodium.

8. Formulation de l'une quelconque des revendications précédentes, où le tampon comprend 15 mM d'acétate de sodium.

9. Formulation de l'une quelconque des revendications précédentes, où la formulation comprend de 165 mM à 185 mM de sucrose.

10. Formulation de l'une quelconque des revendications précédentes, où la formulation comprend de 170 mM à 180 mM de sucrose.

11. Formulation de l'une quelconque des revendications précédentes, où la formulation comprend de 174 mM à 176 mM de sucrose.

12. Formulation de l'une quelconque des revendications précédentes, où la formulation comprend 175 mM de sucrose.

13. Formulation de l'une quelconque des revendications précédentes, où la formulation comprend 0,04 % (v/v) de Polysorbate 20.

14. Formulation de l'une quelconque des revendications précédentes, où la formulation a un pH de 5,8 ou un pH de 5, 6.


### SEQUENCE LISTING

<110> Oncobiologics, Inc.
   TADDEI, Maria
   CHEUNG, Jessica
   GUTKA, Hiten
<120> BUFFERED FORMULATIONS OF BEVACIZUMAB
<130> ONBI-005/001WO
<150> US 62/272,116
   <151> 2015-12-29
<160> 4
<170> Patent In version 3.5
<210> 1
   <211> 453
   <212> PRT
   <213> Artificial Sequence
<220>
   <223> Synthetic Construct
<400> 1
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Thr Asn Tyr
               20                  25                  30
   Gly Met Asn Trp Val Arg Gln Ala Pro Gly Lys Gly Leu Glu Trp Val
           35                  40                  45
   Gly Trp Ile Asn Thr Tyr Thr Gly Glu Pro Thr Tyr Ala Ala Asp Phe
       50                  55                  60
   Lys Arg Arg Phe Thr Phe Ser Leu Asp Thr Ser Lys Ser Thr Ala Tyr
   65                  70                  75                  80
   Leu Gln Met Asn Ser Leu Arg Ala Glu Asp Thr Ala Val Tyr Tyr Cys
                   85                  90                  95
   Ala Lys Tyr Pro His Tyr Tyr Gly Ser Ser His Trp Tyr Phe Asp Val
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Glu Glu Met Thr Lys Asn Gln
           355                 360                 365
   Val Ser Leu Thr Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
       450
<210> 2
   <211> 214
   <212> PRT
   <213> Artificial Sequence
<220>
   <223> Synthetic Construct
<400> 2
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 123
   <212> PRT
   <213> Artificial Sequence
<220>
   <223> Synthetic Construct
<400> 3
<210> 4
   <211> 108
   <212> PRT
   <213> Artificial Sequence
<220>
   <223> Synthetic Construct
<400> 4
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg
               100                 105